# EUROPEAN PATENT APPLICATION

(11) **EP 4 803 877 A1**
(43) Date of publication of application: **09.09.2026**
(21) Application number: 24885874.8
(22) Date of filing: 01.11.2024
(51) Int. Cl.: G01M 99/00, B65G 43/02, G01K 1/02, G01M 13/04, G01N 25/00, G05B 23/02, G06K 7/10, G06K 17/00, G06K 19/07, G06Q 50/10, G08B 25/10

(54) **MONITORING SYSTEM AND MAINTENANCE MANAGEMENT SYSTEM**

(30) Priority: 02.11.2023 JP 2023188435; 02.11.2023 JP 2023188461; 02.11.2023 JP 2023188554; 16.04.2024 JP 2024065897
(71) Applicant: NSK Ltd., Tokyo 141-8560 (JP)
(72) Inventor: KUBOKAWA, Minoru, Fujisawa-shi, Kanagawa 251-8501 (JP); ENDOU, Shigeru, Fujisawa-shi, Kanagawa 251-8501 (JP); KUWAHARA, Masaki, Fujisawa-shi, Kanagawa 251-8501 (JP); TANAKA, Sadayuki, Fujisawa-shi, Kanagawa 251-8501 (JP); KONDOU, Masateru, Fujisawa-shi, Kanagawa 251-8501 (JP); ABE, Daisuke, Fujisawa-shi, Kanagawa 251-8501 (JP)
(74) Representative: SSM Sandmair
(86) International application number: PCT/JP2024/039100
(87) International publication number: WO 2025/095110

(57) **Abstract**

Monitoring is performed appropriately on a monitoring target. A monitoring system includes: a radio tag provided to a monitoring target; and a data acquisition device configured to acquire data from the radio tag. The radio tag includes: a storage configured to store identification information; and a physical quantity sensor configured to detect a physical quantity of the monitoring target and output physical quantity data corresponding to the detected physical quantity. The monitoring system configured to monitor the monitoring target based on the physical quantity data and the identification information acquired by the data acquisition device.

## Description

### Field

The present invention relates to a monitoring system and a maintenance management system.

### Background

There is a known technology to manage items in manufacturing lines by using a Radio Frequency Identification (RFID) tag. For example, in Patent Literature 1, a radio tag stores matching information as to whether the storage-specific information and the display-specific information match each other. Patent Literature 2 discloses a method of tracking assets and inventory by using an RFID tag. In Patent Literature 3, identification information from a radio tag is read by a tag reader device. Patent Literature 4 discloses a technology to perform distribution information management by using an RFID tag. In Patent Literature 5, identification information from a radio tag is read by a tag reader device.

### Citation List

### Patent Literature

Patent Literature 1: JP 2009-26187 A
Patent Literature 2: JP 2011-108239 A
Patent Literature 3: JP 2015-219591 A
Patent Literature 4: JP 2007-089054 A
Patent Literature 5: JP 2008-024385 A

### Summary

### Technical Problem

According to the system described in each of the above documents, it is possible to manage assets and inventory by using an RFID tag and to prevent manufacturing errors and delivery errors due to mix-up of items. However, when a mechanical device that conveys an industrial product along a conveyance direction is a monitoring target, there is room for improvement in performing appropriate monitoring.

The present invention has been made in view of the above, and aims to provide a monitoring system and a maintenance management system capable of appropriately monitoring a monitoring target.

### Solution to Problem

In order to solve the above problem and achieve the above object, a monitoring system includes: a radio tag provided to a monitoring target; and a data acquisition device configured to acquire data from the radio tag. The radio tag includes: a storage configured to store identification information; and a physical quantity sensor configured to detect a physical quantity of the monitoring target and output physical quantity data corresponding to the detected physical quantity. The monitoring system is configured to monitor the monitoring target based on the physical quantity data and the identification information acquired by the data acquisition device.

The physical quantity may be a temperature of the monitoring target, and the physical quantity sensor may be a temperature sensor configured to detect a temperature of the monitoring target and output temperature data corresponding to the detected temperature. The monitoring system may further include a transceiver configured to transmit the temperature data output from the temperature sensor and the identification information. The monitoring system may be configured to monitor the monitoring target based on the temperature data and the identification information acquired by the data acquisition device.

The data acquisition device may be fixed in proximity to the monitoring target.

The monitoring system may further include a plurality of the data acquisition devices, and the radio tag may be provided to each of a plurality of the monitoring targets.

The transceiver may be configured to transmit the temperature data and the identification information in association with each other.

The radio tag may be provided to a bearing. The monitoring system may further include a monitoring terminal device for monitoring the bearing. The monitoring terminal device may include an acquired data storage configured to store data acquired by the data acquisition device. The monitoring terminal device may be configured to monitor the bearing based on the data stored in the acquired data storage.

The physical quantity may be a temperature of the monitoring target, and the physical quantity sensor may be a temperature sensor configured to detect the temperature of the monitoring target and output temperature data corresponding to the detected temperature. The monitoring system may further include: a determination unit configured to determine whether the temperature detected by the temperature sensor exceeds a predetermined threshold; a storage configured to store a determination result and identification information, the determination result having been obtained by the determination unit and indicating that the temperature detected by the temperature sensor exceeds the predetermined threshold; and a transceiver configured to transmit the determination result and the identification information. The data acquisition device may be configured to move in proximity to the radio tag. The monitoring system may be configured to monitor the monitoring target based on the determination result and the identification information acquired from the radio tag by the data acquisition device when the data acquisition device moves in proximity to the radio tag.

The monitoring system may further include a plurality of the data acquisition devices. The radio tag may be provided to each of a plurality of the monitoring targets. The data acquisition devices may be configured to store the identification information acquired from the radio tags when the data acquisition devices move in proximity to the radio tags.

The transceiver may be configured to transmit the determination result and the identification information in association with each other.

The radio tag may be provided to a bearing. The monitoring system may further include a monitoring terminal device for monitoring the bearing. The monitoring terminal device may include an acquired data storage configured to store data acquired by the data acquisition device, and the monitoring terminal device may be configured to monitor the bearing based on the data stored in the acquired data storage.

The monitoring terminal device may further include an alarm configured to output an alarm based on the determination result.

The physical quantity may be acceleration of the monitoring target, and the physical quantity sensor may be an acceleration sensor configured to detect the acceleration of the monitoring target. The monitoring system may further include: a determination unit configured to determine whether vibration based on the acceleration detected by the acceleration sensor exceeds a predetermined threshold; a storage configured to store a determination result and identification information, the determination result having been obtained by the determination unit and indicating that the vibration based on the acceleration detected by the acceleration sensor exceeds the predetermined threshold; and a transceiver configured to transmit the determination result and the identification information. The data acquisition device may be configured to move in proximity to the radio tag. The monitoring system may be configured to monitor the monitoring target based on the determination result and the identification information acquired from the radio tag by the data acquisition device when the data acquisition device moves in proximity to the radio tag.

The monitoring system may further include a plurality of the data acquisition devices. The radio tag may be provided to each of a plurality of the monitoring targets. The data acquisition devices may be configured to store the identification information acquired from the radio tags when the data acquisition devices move in proximity to the radio tags.

The transceiver may be configured to transmit the determination result and the identification information in association with each other.

The radio tag may be provided to a bearing. The monitoring system may further include a monitoring terminal device for monitoring the bearing. The monitoring terminal device may include an acquired data storage configured to store data acquired by the data acquisition device, and the monitoring terminal device may be configured to monitor the bearing based on the data stored in the acquired data storage.

The monitoring terminal device may further include an alarm configured to output an alarm based on the determination result.

A maintenance management system according to an aspect of the present disclosure includes: a plurality of radio tags provided in proximity to a plurality of maintenance management targets and each including a temperature sensor configured to acquire a temperature of the corresponding maintenance management target; a data acquisition device configured to acquire f temperature data values from the radio tags; a storage configured to store the temperature data values acquired by the data acquisition device; a determination unit configured to determine whether the temperature data value stored in the storage is equal to or greater than a predetermined threshold; and a display configured to display the temperature data values read from the storage. Based on a determination result obtained by the determination unit, the display displays a temperature data value indicating the temperature equal to or higher than the predetermined threshold, among the temperature data values, in a mode different from the other temperature data values.

Based on a determination result obtained by the determination unit, when the temperature data value indicates a value equal to or greater than a first threshold, the display may display the temperature data value in a mode different from the other temperature data values.

Based on a determination result obtained by the determination unit, when a difference between the temperature data values of maintenance management targets at adjacent positions is equal to or greater than a second threshold, the display may display the higher temperature data value thereof in a mode different from the other temperature data value thereof.

The maintenance management system may further include a calculator configured to calculate an average value. Based on a determination result obtained by the determination unit, when a difference between the average value and the temperature data value is equal to or greater than a third threshold, the display may display the temperature data value in a mode different from the other temperature data values.

The display may be configured to display the temperature data values by a bar chart. Among the temperature data values to be displayed by the bar chart, the temperature data value to be displayed in a mode different from the other temperature data values may be displayed in a color different from a display color of the other temperature data values.

### Advantageous Effects of Invention

According to the monitoring system of the present disclosure, the monitoring target can be appropriately monitored. In addition, the present disclosure can facilitate determination as to whether the temperature acquired from the radio tag is an appropriate value.

### Brief Description of Drawings

FIG. 1 is a diagram illustrating a monitoring system according to a first embodiment of the present disclosure.
FIG. 2 is a diagram illustrating an example of a monitoring target of a monitoring system.
FIG. 3 is a perspective view illustrating an example of a mechanical component in FIG. 2.
FIG. 4 is a diagram illustrating an example in which a plurality of mechanical devices are set as monitoring targets.
FIG. 5 is a flowchart illustrating an operation example of the monitoring system according to the first embodiment.
FIG. 6 is a diagram illustrating an example of data acquired from a radio tag by the tag reader device.
FIG. 7 is a diagram illustrating an example of a temperature measurement result of each shaft member.
FIG. 8 is a diagram illustrating an example of a temperature measurement result of one shaft member.
FIG. 9 is a diagram illustrating an example of a temperature measurement result of one shaft member.
FIG. 10 is a diagram illustrating an example of a temperature measurement result of one shaft member.
FIG. 11 is a diagram illustrating a monitoring system according to a second embodiment of the present disclosure.
FIG. 12 is a flowchart illustrating an operation example of the monitoring system according to the second embodiment.
FIG. 13 is a diagram illustrating a monitoring system according to a third embodiment of the present disclosure.
FIG. 14 is a diagram illustrating a configuration example of a determination unit in FIG. 13.
FIG. 15 is a diagram illustrating an example in which a plurality of mechanical devices are set as monitoring targets.
FIG. 16 is a flowchart illustrating an operation example of the monitoring system according to the third embodiment.
FIG. 17 is a diagram illustrating an example of data acquired from a radio tag by the tag reader device.
FIG. 18 is a diagram illustrating a tag reader device of a monitoring system according to a fourth embodiment of the present disclosure.
FIG. 19 is a diagram illustrating a monitoring system according to a fifth embodiment of the present disclosure.
FIG. 20 is a diagram illustrating a configuration example of a determination unit in FIG. 19.
FIG. 21 is a diagram illustrating an example of data acquired from a radio tag by the tag reader device.
FIG. 22 is a diagram illustrating a maintenance management system according to a seventh embodiment of the present disclosure.
FIG. 23 is a diagram illustrating an example of a maintenance management target of the maintenance management system.
FIG. 24 is a flowchart illustrating an operation example of the maintenance management system according to the seventh embodiment.
FIG. 25 is a flowchart illustrating a first example of processing in a controller of the maintenance management device in FIG. 22.
FIG. 26 is a diagram illustrating an example of data acquired from a radio tag by the tag reader device.
FIG. 27 is a flowchart illustrating a second example of processing in a controller of the maintenance management device in FIG. 22.
FIG. 28 is a diagram illustrating a maintenance management system according to a ninth embodiment of the present disclosure.
FIG. 29 is a flowchart illustrating a third example of processing in a controller of the maintenance management device in FIG. 28.

### Description of Embodiments

Hereinafter, embodiments of the present invention will be described in detail with reference to the drawings. In the following description of each embodiment, the same or equivalent components as those of other embodiments are denoted by the same reference numerals, and the description thereof will be simplified or omitted. The present invention is not limited by each embodiment. In addition, the constituent elements of each embodiment include those that can be easily replaced by those skilled in the art or those that are substantially the same. The configurations in the following can be appropriately combined with each other. The configuration can be omitted, replaced, or changed without departing from the scope and spirit of the invention. In a second embodiment and subsequent embodiments, description of matters in common with a first embodiment may be appropriately omitted.

### (First Embodiment)

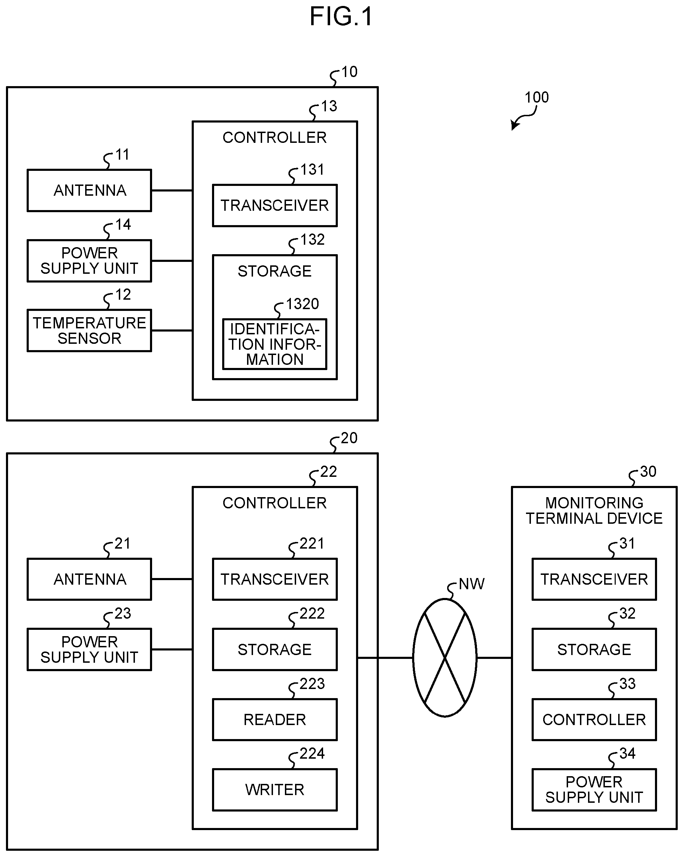

FIG. 1 is a diagram illustrating a monitoring system according to the first embodiment of the present disclosure. In FIG. 1, a monitoring system 100 includes a radio tag 10, a tag reader device 20, and a monitoring terminal device 30. The radio tag 10 is provided on a monitoring target of the monitoring system 100. The tag reader device 20 can acquire data from the radio tag 10. In addition, the tag reader device 20 can write data into the radio tag 10. The tag reader device 20 corresponds to a data acquisition device of the present disclosure.

### (Radio Tag)

The radio tag 10 includes an antenna 11, a temperature sensor 12, a controller 13, and a power supply unit 14. The controller 13 includes a transceiver 131 and a storage 132. The radio tag 10 is an RFID tag, for example.

The antenna 11 is a transmission/reception antenna. That is, the antenna 11 has a function as a transmission antenna and a function as a reception antenna.

The temperature sensor 12 detects a temperature. Specifically, the temperature sensor 12 detects the temperature of the monitoring target on which the radio tag 10 is provided. The temperature detected by the temperature sensor 12 is stored in the storage 132 of the controller 13 as temperature data. That is, the temperature sensor 12 outputs temperature data corresponding to the temperature.

The transceiver 131 can wirelessly receive data via the antenna 11. The transceiver 131 can wirelessly transmit data via the antenna 11.

The storage 132 stores identification information 1320 for identifying the radio tag 10 itself. In addition, the storage 132 stores the temperature detected by the temperature sensor 12 as temperature data. The data stored in the storage 132 can be read.

The power supply unit 14 supplies power to each component in the radio tag 10. The power supply unit 14 is a primary battery, for example. Since power is supplied from the power supply unit 14, the radio tag 10 can perform detection of the temperature by the temperature sensor 12 and store temperature data in the storage 132.

When a data read signal is transmitted from the tag reader device 20, the radio tag 10 reads data stored in the storage 132 and transmits the read data to the tag reader device 20. At this time, the transceiver 131 of the radio tag 10 transmits the temperature data and the identification information to the tag reader device 20 in association with each other.

### (Tag Reader Device)

The tag reader device 20 includes an antenna 21, a controller 22, and a power supply unit 23.

The antenna 21 is a transmission/reception antenna. That is, the antenna 21 has a function as a transmission antenna and a function as a reception antenna.

The controller 22 includes a transceiver 221, a storage 222, a reader 223, and a writer 224. The transceiver 221 can wirelessly transmit and receive data to and from the radio tag 10 via the antenna 21. In addition, the transceiver 221 can transmit and receive data to and from the monitoring terminal device 30 via the network NW. The tag reader device 20 can transmit temperature data of the temperature sensor 12 of the radio tag 10 to the monitoring terminal device 30.

The storage 222 stores the data acquired by the transceiver 221. The storage 222 stores the temperature data and the identification information acquired by the transceiver 221 in association with each other. In addition, the storage 222 stores various data needed for the operation of the tag reader device 20.

The reader 223 can receive data transmitted from the radio tag 10 using the antenna 21 and the transceiver 221 and can read data stored in the radio tag 10. With this configuration, the tag reader device 20 can acquire data from the radio tag 10.

The reader 223 can perform wireless communication simultaneously with a plurality of the radio tags 10, and can simultaneously acquire temperature data from the plurality of radio tags 10. At this time, the temperature data associated with the identification information is acquired. Accordingly, the tag reader device 20 acquires temperature data of each of the temperature sensors of the plurality of radio tags 10 in a relatively short time. The tag reader device 20 transmits temperature data of each of the plurality of temperature sensors to the monitoring terminal device 30.

The writer 224 can transmit data to the radio tag 10 using the antenna 21 and the transceiver 221. With this configuration, the tag reader device 20 can write data into the radio tag 10.

The power supply unit 23 supplies power to each component in the tag reader device 20. The power supply unit 23 is a primary battery, for example.

### (Monitoring Terminal Device)

The monitoring terminal device 30 includes a transceiver 31, a storage 32, a controller 33, and a power supply unit 34. The transceiver 31 can transmit and receive data to and from the tag reader device 20 via the network NW. The monitoring terminal device 30 may be provided in proximity to the tag reader device 20 or may be provided at a remote place.

The storage 32 stores, for example, data acquired by the tag reader device 20 from the radio tag 10. The storage 32 corresponds to an acquired data storage of the present disclosure. The controller 33 includes, for example, a Central Processing Unit (CPU), Read Only Memory (ROM), Random Access Memory (RAM), an input interface, and an output interface, which are not illustrated. The CPU, the ROM, and the RAM (which are not illustrated) are connected to each other by an internal bus. The ROM stores programs such as BIOS. The CPU implements various functions by executing a program stored in the ROM or the storage 32 while using the RAM as a work area. The controller 33 can edit the data stored in the storage 32, such as classification and rearrangement. The power supply unit 34 supplies power to each component of the monitoring terminal device 30.

### (Example of Monitoring Target)

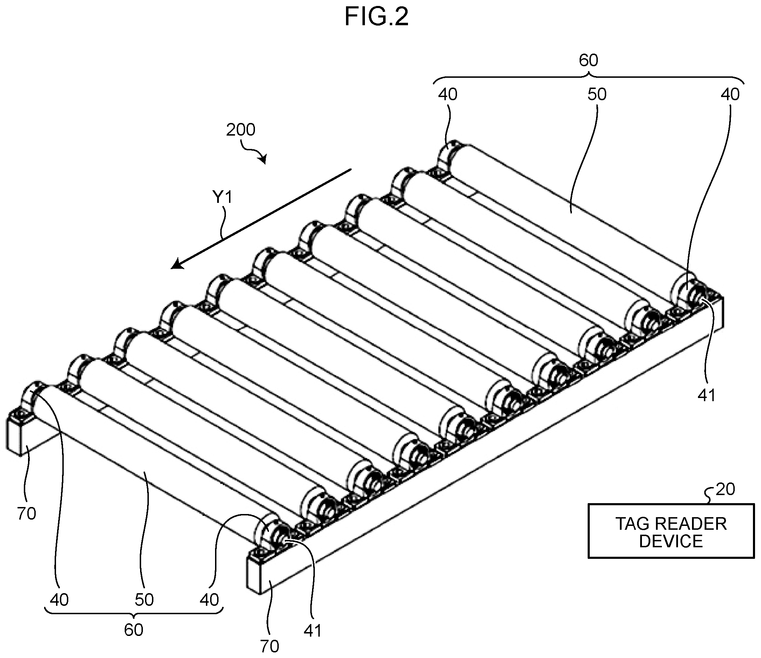

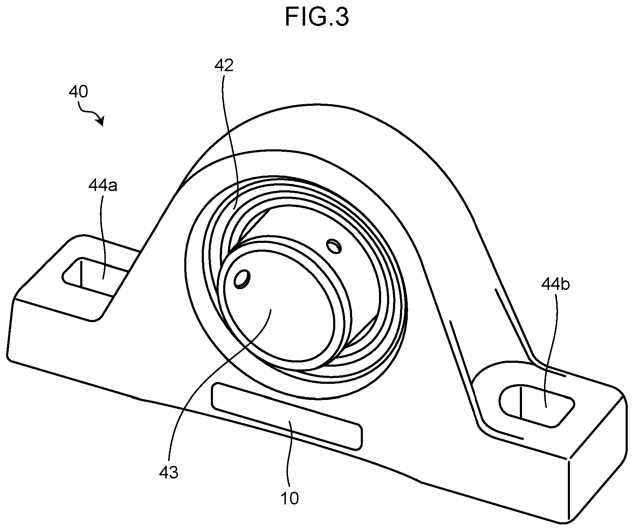

FIG. 2 is a diagram illustrating an example of a monitoring target of the monitoring system. FIG. 2 is a diagram illustrating a case where a mechanical device 200 is set as the monitoring target. FIG. 3 is a perspective view illustrating an example of a mechanical component 40 in FIG. 2.

In FIG. 2, the mechanical device 200 is, for example, a roller conveyor that conveys an industrial product (not illustrated) along a conveyance direction. The mechanical device 200 includes a pair of support bases 70 and a plurality of roller devices 60. In the present embodiment, the number of the roller devices 60 is ten, but is not limited to this number.

The pair of support bases 70 supports the plurality of roller devices. The pair of support bases 70 has a rectangular parallelepiped shape extending along the direction of arrow Y1, which is the conveyance direction of the industrial product.

The roller device 60 includes a roller member 50 and a pair of mechanical components 40. The mechanical component 40 is the monitoring target of the present disclosure.

The roller device 60 includes a shaft member 41 and a roller member 50. The shaft member has a columnar shape extending along the central axis.

The roller member 50 is a cylindrical shape member disposed on the circumferential side surface of the shaft member 41, and rotates integrally with the shaft member 41. Both ends of the shaft member 41 are exposed from the roller member 50.

The pair of mechanical components supports the roller member 50 so as to be rotatable relative thereto. Specifically, the pair of mechanical components supports both ends of the shaft member so as to be rotatable relative thereto. The mechanical component 40 is a plummer block, for example.

As illustrated in FIG. 2, the tag reader device 20 is provided in proximity to the mechanical device 200. The tag reader device 20 is provided at the position where the tag reader device can transmit and receive signals to and from the radio tags 10. The tag reader device 20 is fixed to a place such as a ceiling, a wall surface, a column, or the like of a room in which the mechanical device 200 is installed, for example.

As illustrated in FIG. 3, the mechanical component 40 includes a bearing 42 and holes 44a and 44b. The end of the shaft member 41 of the roller member 50 is inserted into a through hole 43 of the bearing 42. The bearing 42 rotatably supports the shaft member 41. The mechanical component 40 is fixed to the support base 70 (refer to FIG. 2) by, for example, bolts (not illustrated). The bolts (not illustrated) pass through the holes 44a and 44b, for example, and are inserted into the screw holes of the support base 70.

The mechanical component 40 is provided with the radio tag 10. The radio tag 10 is provided on the lower side of the bearing 42, for example. The radio tag 10 is attached to the surface of the mechanical component 40 by an adhesive tape, for example. The radio tag 10 includes the temperature sensor 12 as described above. The temperature data detected by the temperature sensor 12 is stored in the storage 132 in the radio tag 10, whereby the temperature data is transmitted to the tag reader device 20 together with the identification information 1320.

In the example illustrated in FIG. 2, one tag reader device 20 is provided for one mechanical device 200. One mechanical device 200 includes ten roller members 50. The radio tags 10 are individually attached to twenty mechanical components 40 provided at both ends of the roller members 50. In this example, all the radio tags 10 of the twenty mechanical components 40 are located in a communicable area of the tag reader device 20. Accordingly, the tag reader device 20 can acquire the identification information and the temperature data from each of the twenty radio tags 10 provided on the twenty mechanical components 40.

Another configuration may be employed in which the radio tags 10 are provided on the mechanical components 40 on one end side and no radio tags 10 are provided on the mechanical components 40 on the other end side, out of the both end sides of the roller members 50. Some of the mechanical components 40 in the mechanical device 200 may be provided with the radio tag 10 and may be set as the monitoring target.

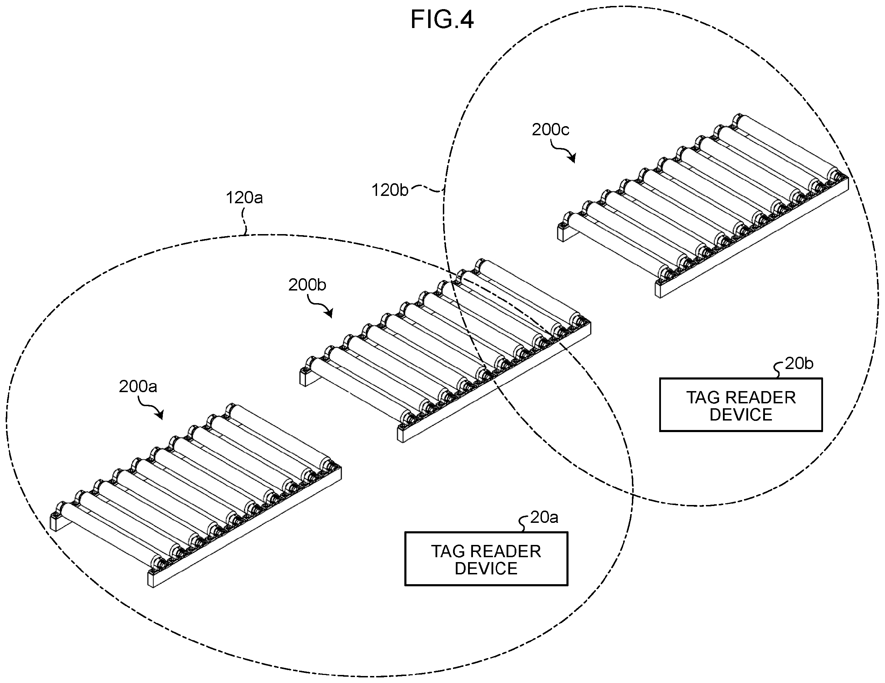

FIG. 4 is a diagram illustrating an example in which a plurality of the mechanical devices 200 are set as monitoring targets. In the example illustrated in FIG. 4, three mechanical devices 200a, 200b, and 200c are set as the monitoring targets. In the example illustrated in FIG. 4, two tag reader devices 20a and 20b are provided for three mechanical devices 200a, 200b, and 200c. The two tag reader devices 20a and 20b are fixed to a place such as a ceiling, a wall surface, a column, or the like of a room in which the mechanical devices 200a, 200b, and 200c are installed, for example.

Here, in a communicable area 120a of the tag reader device 20a, all the mechanical components 40 included in the mechanical device 200a and some of the mechanical components 40 included in the mechanical device 200b are located. On the other hand, in a communicable area 120b of the tag reader device 20b, all the mechanical components 40 included in the mechanical device 200c and some of the mechanical components 40 included in the mechanical device 200b are located. In this manner, by arranging each component such that all the mechanical components 40 are provided in an area obtained by combining the communicable area 120a and the communicable area 120b, the tag reader devices 20a and 20b fixed to a ceiling, a wall surface, a column, or the like can acquire the identification information and the temperature data from each radio tag 10.

Referring back to FIG. 1, the identification information and the temperature data acquired from each radio tag 10 are stored in the storage 222 and then transmitted to the monitoring terminal device 30 via the network NW. The monitoring terminal device 30 stores the identification information and the temperature data in the storage 32. The controller 33 can edit the data stored in the storage 32, such as classification and rearrangement.

The detection of the temperature by the temperature sensor 12 of each radio tag 10 is performed, for example at a predetermined interval. For example, the temperature sensor 12 detects the temperature once a day at a predetermined time. Alternatively, for example, the detection may be performed every preset time. For example, the temperature sensor 12 may detect the temperature every 1 hour, every 30 minutes, every 1 minute, and every 30 seconds.

The identification information and the temperature data may be transmitted to the tag reader device 20 every time the temperature is detected by the radio tag 10, or may be collectively transmitted to the tag reader device 20 when the amount of data stored in the storage 132 of the radio tag 10 reaches a predetermined amount. In the former case, the monitoring process can be performed more promptly. In the latter case, the consumption of the power supply unit 14 can be further reduced by collectively transmitting the data.

### (Operation Example)

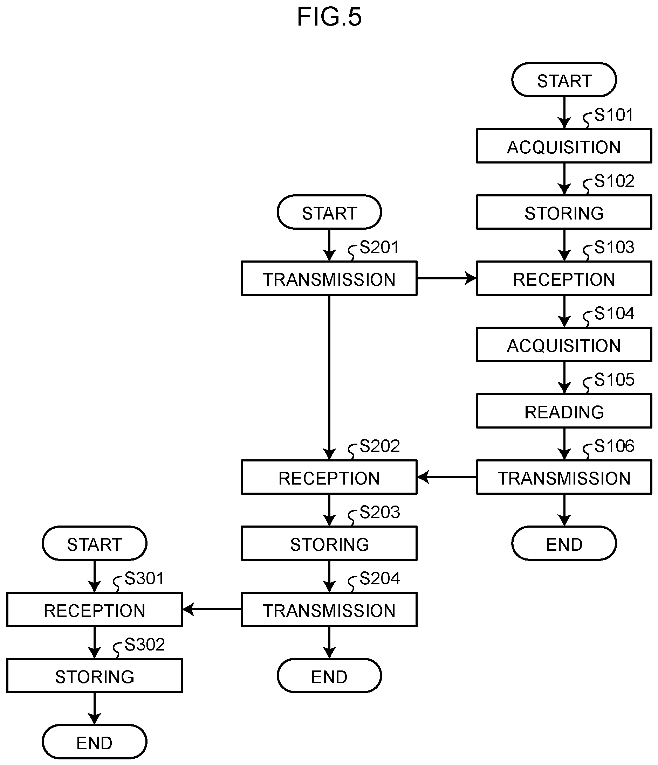

FIG. 5 is a flowchart illustrating an operation example of the monitoring system 100 according to the first embodiment. FIG. 5 illustrates operations of the radio tag 10, the tag reader device 20, and the monitoring terminal device 30 of the monitoring system 100.

In FIG. 5, steps S101 to S106 illustrate an operation example of the radio tag 10, steps S201 to S204 illustrate an operation example of the tag reader device 20, and steps S301 to S302 illustrate an operation example of the monitoring terminal device 30.

In FIG. 5, the radio tag 10 acquires temperature data from the temperature sensor 12 in advance (step S101), and stores the temperature data in the storage 132 (step S102).

Thereafter, when a data read signal is transmitted from the tag reader device 20 to the radio tag 10 (step S201), the radio tag 10 receives the read signal (step S103). Then, the radio tag 10 acquires temperature data from the temperature sensor 12 (step S104) and reads identification information stored in the storage 132 (step S105). The radio tag 10 transmits the temperature data together with the identification information (step S106), and the tag reader device 20 receives the temperature data and the identification information (step S202).

The tag reader device 20 stores the received temperature data and identification information in the storage 222 (step S203). Thereafter, the tag reader device 20 transmits the temperature data and the identification information (step S204), and the monitoring terminal device 30 receives the temperature data and the identification information (step S301). The monitoring terminal device 30 stores the received temperature data and identification information in the storage 32 (step S302). The monitoring terminal device 30 can acquire the temperature data and the identification information by the above processing, and can edit the data stored in the storage 32, such as classification and rearrangement. The monitoring target can be monitored by utilizing the data stored in the storage 32.

### (Example of Temperature Data)

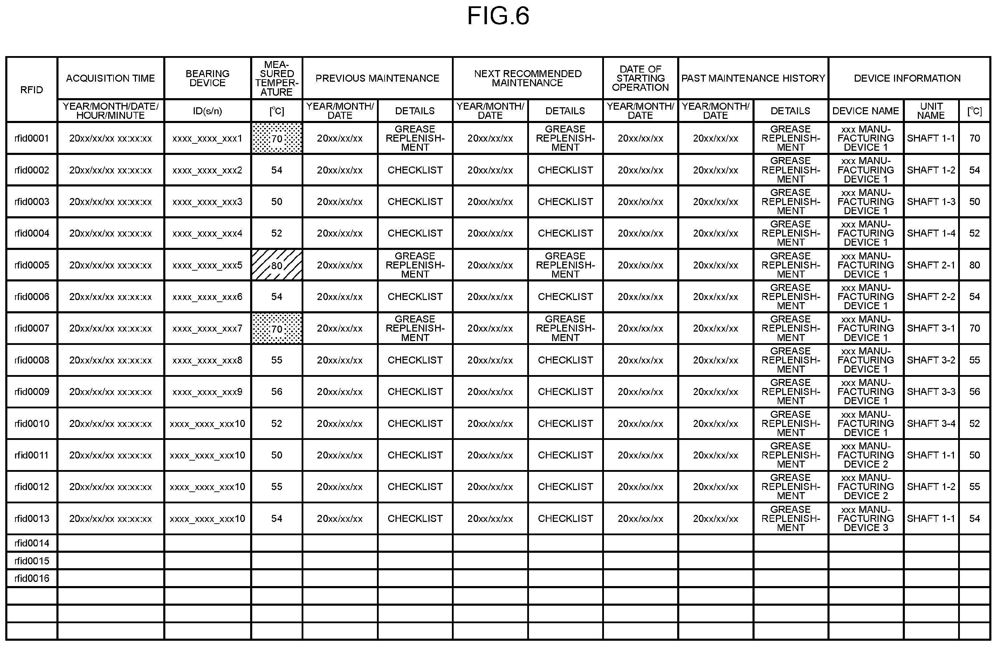

FIG. 6 is a diagram illustrating an example of data acquired from the radio tag 10 by the tag reader device 20. FIG. 6 illustrates an example of data transmitted from the tag reader device 20 to the monitoring terminal device 30 and stored in the storage 32.

As illustrated in FIG. 6, identification information "rfid0001", "rfid0002",... regarding the RFID is stored in the storage 32 in association with other data items. For example, the identification information "rfid0001" is associated with acquisition time (namely, year/month/date/hour/minute), the serial number (s/n) as the identification information (ID) of the bearing, and the measured temperature based on the temperature data. In this example, the identification information is further associated with the year/month/date and details of the previous maintenance (for example, grease replenishment, registration to checklist), the year/month/date and details of next recommended maintenance, the year/month/date of the operation start, the year/month/date and details of the past maintenance history, the device name as device information, the unit name, and the measured temperature.

The unit name is information that identifies the shaft member described with reference to FIGS. 2 and 4. For example, these are "shaft 1-1", "shaft 1-2", "shaft 1-3", "shaft 1-4", "shaft 2-1", "shaft 2-2", "shaft 3-1", "shaft 3-2", "shaft 3-3", and "shaft 3-4".

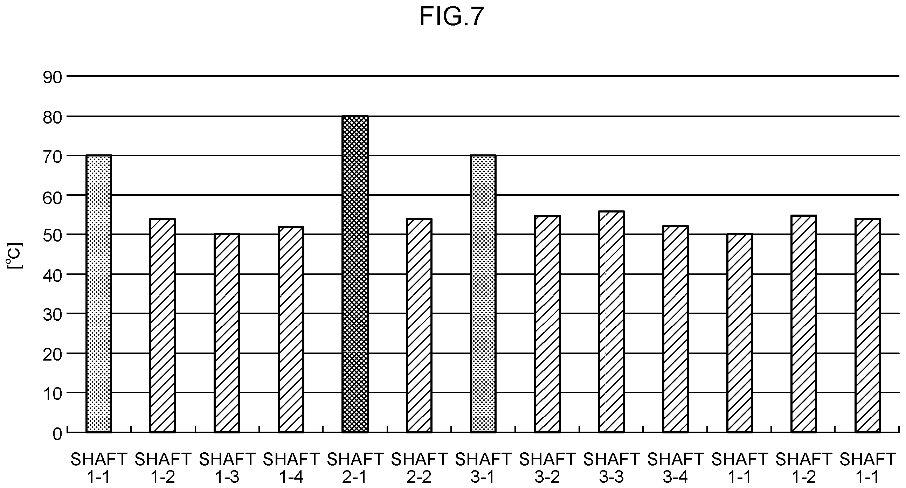

FIG. 7 is a diagram illustrating an example of a temperature measurement result of each shaft member. The monitoring terminal device 30 can edit each data item illustrated in FIG. 6 and display the graph illustrated in FIG. 7 on a screen (not illustrated).

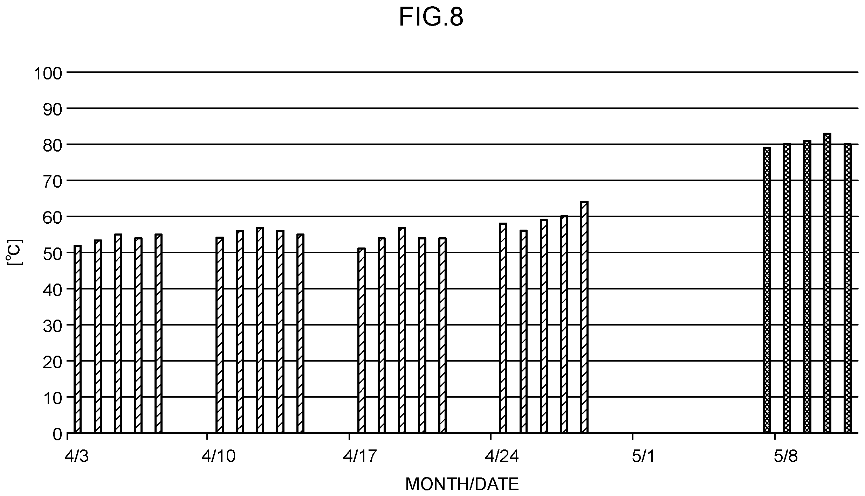

FIGS. 8 to 10 are diagrams illustrating examples of temperature measurement results of one shaft member. FIGS. 8 to 10 are diagrams illustrating examples of temperature measurement results of shaft 2-1. FIG. 8 illustrates an average value of temperature data for a certain period (for example, a whole day) for shaft 2-1. In the example illustrated in FIG. 8, temperature measurement is not performed on a non-operating day such as "May 1", and thus, there is no temperature data for the non-operating day.

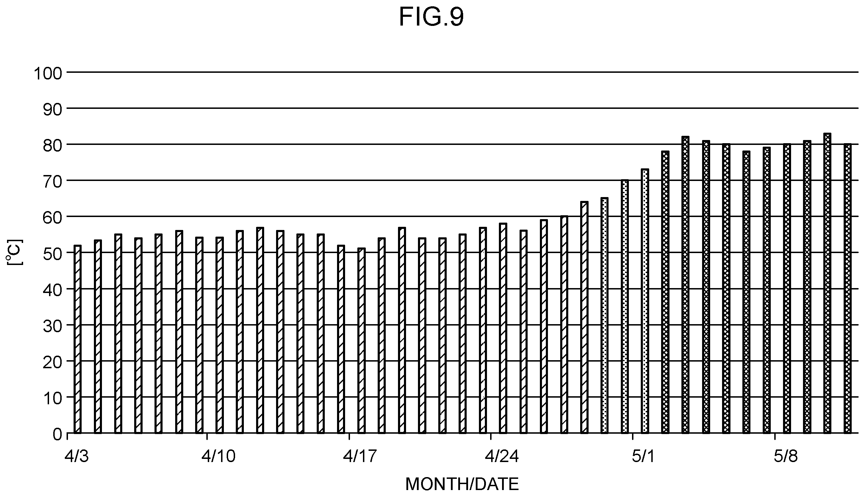

FIG. 9 illustrates an average value of temperature data in a certain period (for example, a whole day) for shaft 2-1. In the example illustrated in FIG. 9, temperature measurement is performed even on a non-operating day such as "May 1", and thus, there is temperature data even for the non-operating day.

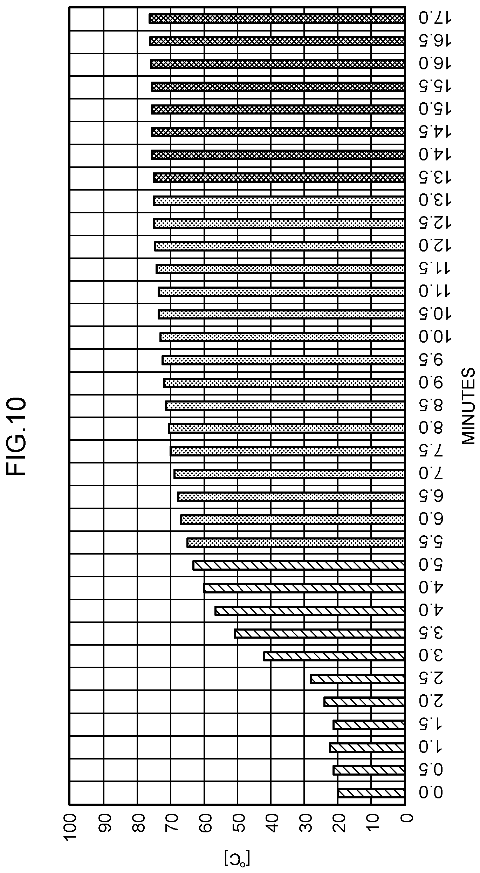

FIG. 10 illustrates the time series of temperature data for shaft 2-1. The example illustrated in FIG. 10 indicates a result of measurements performed every 30 seconds. In this manner, the frequency of measurement can be increased. The frequency of measurement may be increased for the shaft registered in the checklist.

### (Second Embodiment)

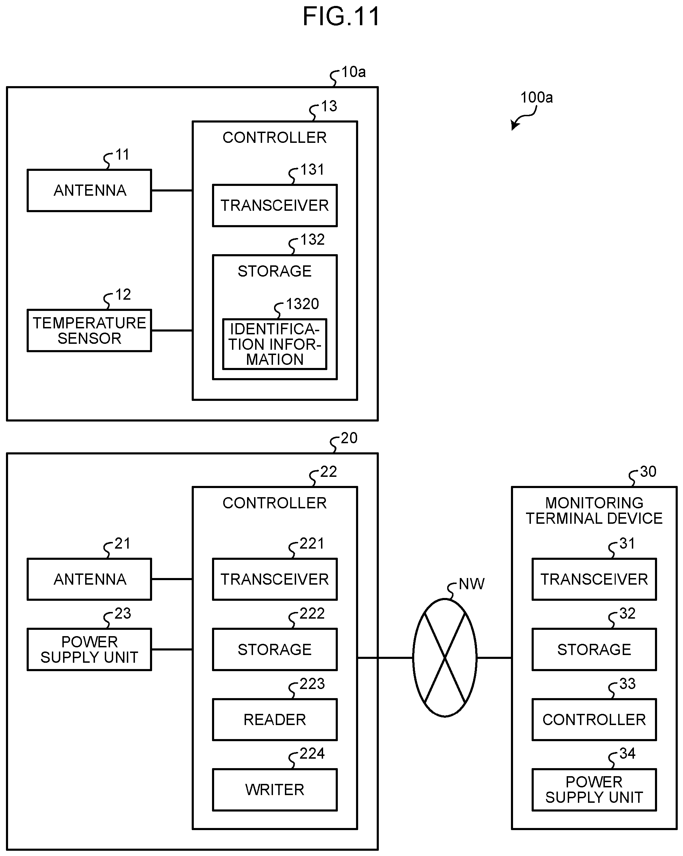

FIG. 11 is a diagram illustrating a monitoring system according to a second embodiment of the present disclosure. In FIG. 10, a monitoring system 100a according to the second embodiment is different from the monitoring system 100 according to the first embodiment in that the monitoring system 100a includes a radio tag 10a not equipped with a power supply unit. The radio tag 10a operates on power based on an electromagnetic wave transmitted by the tag reader device 20. Specifically, the antenna 11 of the radio tag 10a receives the electromagnetic wave transmitted from the tag reader device 20, and then, the received electromagnetic wave induces a current. The radio tag 10a operates using this current as a power source.

In the monitoring system according to the first embodiment described above, the detection of the temperature by the temperature sensor 12 of each radio tag 10 is performed at a predetermined interval, for example, and the detected temperature is sequentially stored in the storage 132. When a data read signal is transmitted from the tag reader device 20 to the radio tag 10, the stored temperature data and identification information are transmitted from the radio tag 10 to the tag reader device 20.

In contrast, in the monitoring system 100a according to the second embodiment, the temperature sensor 12 detects the temperature when the data read signal is transmitted from the tag reader device 20 to the radio tag 10. That is, the radio tag 10 operates on the power based on the electromagnetic wave of the read signal, and detects the temperature by the temperature sensor 12. Other operations of the monitoring system 100a are similar to those of the monitoring system 100 according to the first embodiment.

### (Operation Example)

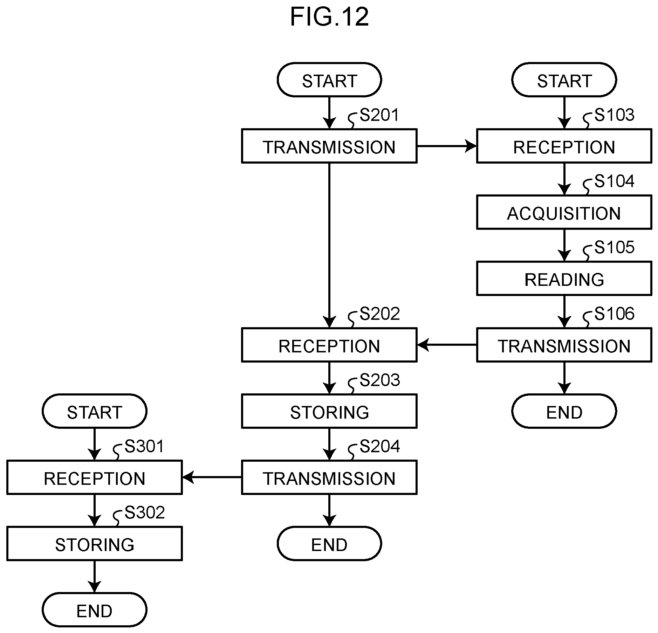

FIG. 12 is a flowchart illustrating an operation example of the monitoring system 100a according to the second embodiment. FIG. 12 illustrates operations of the radio tag 10, the tag reader device 20, and the monitoring terminal device 30 of the monitoring system 100a.

In FIG. 12, steps S103 to S106 illustrate an operation example of the radio tag 10, steps S201 to S204 illustrate an operation example of the tag reader device 20, and steps S301 to S302 illustrate an operation example of the monitoring terminal device 30.

In FIG. 12, when a data read signal is transmitted from the tag reader device 20 to the radio tag 10 (step S201), the radio tag 10 receives the read signal (step S103). Then, the radio tag 10 acquires temperature data from the temperature sensor 12 (step S104), and reads the temperature data and the identification information stored in the storage 132 (step S105). The radio tag 10 transmits the temperature data together with the identification information (step S106), and the tag reader device 20 receives the temperature data and the identification information (step S202).

The subsequent operation is similar to the operation of the monitoring system 100 described with reference to FIG. 5. That is, the tag reader device 20 stores the received temperature data and identification information in the storage 222 (step S203). Thereafter, the tag reader device 20 transmits the temperature data and the identification information (step S204), and the monitoring terminal device 30 receives the temperature data and the identification information (step S301). The monitoring terminal device 30 stores the received temperature data and identification information in the storage 32 (step S302). The monitoring terminal device 30 can acquire the temperature data and the identification information by the above processing, and can edit the data stored in the storage 32, such as classification and rearrangement. The monitoring target can be monitored by utilizing the data stored in the storage 32.

### (Summarization of First Embodiment and Second Embodiment)

According to the monitoring system 100 of the first embodiment or the monitoring system 100a of the second embodiment described above, the production facility can be monitored in the production facility such as the site of the factory without the need for the maintenance inspector to directly visit the place in proximity to the mechanical facility. Specifically, it is possible to detect a sign of abnormality associated with temperature change and identify the bearing. For example, temperature monitoring and processing can be performed in a remote control room. In each of the above embodiments, the bearing is a monitoring target, but the target is not limited to this and, for example, shaft extension by heat, motor rated operation, and the like can also be monitored. Furthermore, for example, in a case where a bearing and a box for packing the bearing are shipped together with a tag, it is also possible to monitor detection of an abnormal temperature during a period from shipment to assembly onto a mechanical device in the course of movement.

### (Third Embodiment)

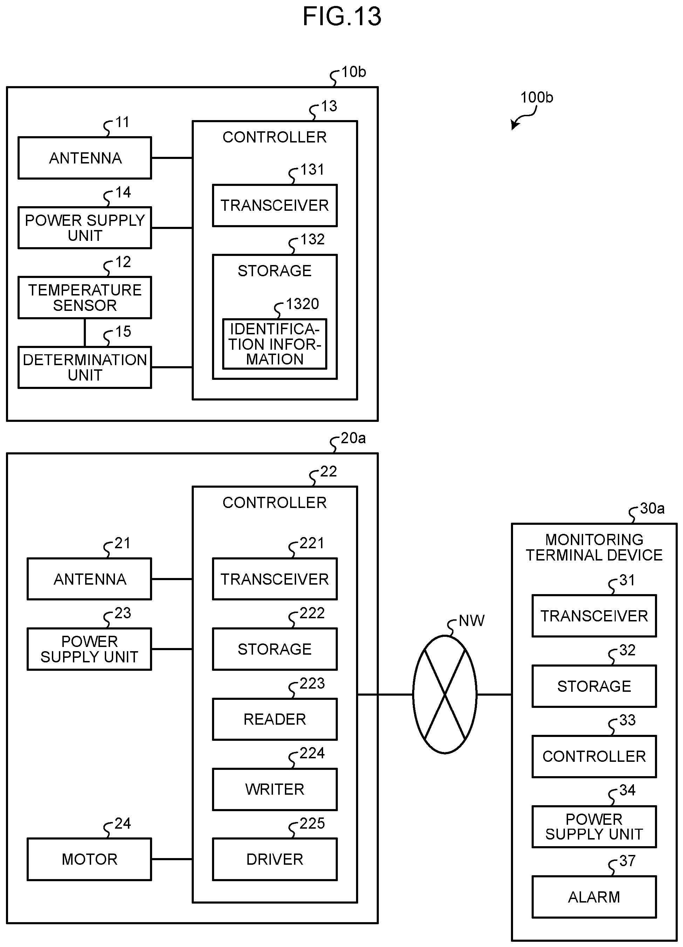

FIG. 13 is a diagram illustrating a monitoring system according to a third embodiment of the present disclosure. In FIG. 13, a monitoring system 100b includes a radio tag 10b, a tag reader device 20a, and a monitoring terminal device 30a. The radio tag 10b is provided on a monitoring target of the monitoring system 100b. The tag reader device 20a can acquire data from the radio tag 10b. In addition, the tag reader device 20a can write data into the radio tag 10b. The tag reader device 20a corresponds to a data acquisition device of the present disclosure.

### (Radio Tag)

The radio tag 10b includes an antenna 11, a temperature sensor 12, a controller 13, a power supply unit 14, and a determination unit 15. The controller 13 includes a transceiver 131 and a storage 132. The radio tag 10b is an RFID tag, for example.

The antenna 11 is a transmission/reception antenna. That is, the antenna 11 has a function as a transmission antenna and a function as a reception antenna.

The temperature sensor 12 detects a temperature. Specifically, the temperature sensor 12 detects the temperature of the monitoring target on which the radio tag 10b is provided. The temperature detected by the temperature sensor 12 is transmitted to the determination unit 15.

The transceiver 131 can wirelessly receive data via the antenna 11. The transceiver 131 can wirelessly transmit data via the antenna 11.

The storage 132 stores identification information 1320 for identifying the radio tag 10b itself. In addition, the storage 132 stores the determination result obtained by the determination unit 15 as data. The data stored in the storage 132 can be read.

The power supply unit 14 supplies power to each component in the radio tag 10b. The power supply unit 14 is a primary battery, for example. Since power is supplied from the power supply unit 14, the radio tag 10b can perform detection of the temperature using the temperature sensor 12 and store the temperature determination result based on the temperature data in the storage 132.

The determination unit 15 determines whether the detected value of the temperature output from the temperature sensor 12 exceeds a predetermined threshold. The determination result obtained by the determination unit 15 is stored in the storage 132. For example, in a case where the detection value of the temperature output from the temperature sensor 12 exceeds the predetermined threshold, the data of the determination result obtained by the determination unit 15 is "1", and in a case where the detection value is equal to or less than the predetermined threshold, the data of the determination result obtained by the determination unit 15 is "0", and accordingly, "1" or "0" as the determination result is stored in the storage 132.

The threshold for the detected value of temperature is set as follows, for example. That is, the temperature in the absence of abnormality in the mechanical device 200 is measured in advance, and a value slightly exceeding the value of the measured temperature is set as the threshold.

When a data read signal is transmitted from the tag reader device 20a, the radio tag 10b reads data stored in the storage 132 and transmits the data to the tag reader device 20a. At this time, the transceiver 131 of the radio tag 10b transmits the temperature determination result and the identification information to the tag reader device 20a in association with each other.

### (Tag Reader Device)

The tag reader device 20a includes an antenna 21, a controller 22, a power supply unit 23, and a motor 24.

The antenna 21 is a transmission/reception antenna. That is, the antenna 21 has a function as a transmission antenna and a function as a reception antenna.

The controller 22 includes a transceiver 221, a storage 222, a reader 223, a writer 224, and a driver 225. The transceiver 221 can wirelessly transmit and receive data to and from the radio tag 10b via the antenna 21. In addition, the transceiver 221 can transmit and receive data to and from the monitoring terminal device 30 via the network NW. The tag reader device 20a can transmit a temperature determination result based on temperature data of the temperature sensor 12 of the radio tag 10b to the monitoring terminal device 30.

The storage 222 stores the data acquired by the transceiver 221. The storage 222 stores the temperature determination result and the identification information acquired by the transceiver 221 in association with each other. In addition, the storage 222 stores various data and programs needed for the operation of the tag reader device 20a.

The reader 223 can receive data transmitted from the radio tag 10b using the antenna 21 and the transceiver 221 and can read data stored in the radio tag 10b. With this configuration, the tag reader device 20a can acquire data from the radio tag 10b.

The reader 223 can perform wireless communication simultaneously with the plurality of radio tags 10b, and can simultaneously acquire temperature determination results from the plurality of radio tags 10b. At this time, the temperature determination result associated with the identification information is acquired. Accordingly, the tag reader device 20a acquires the temperature determination result based on the temperature data of each of the temperature sensors of the plurality of radio tags 10b in a relatively short time. The tag reader device 20a transmits the temperature determination result based on temperature data of a plurality of temperature sensors to the monitoring terminal device 30.

The writer 224 can transmit data to the radio tag 10b using the antenna 21 and the transceiver 221. With this configuration, the tag reader device 20a can write data into the radio tag 10b. The driver 225 controls the motor 24 to move the tag reader device 20a.

The power supply unit 23 supplies power to each component in the tag reader device 20a. The power supply unit 23 is a primary battery, for example.

### (Monitoring Terminal Device)

The monitoring terminal device 30 includes a transceiver 31, a storage 32, a controller 33, a power supply unit 34, and an alarm 37. The transceiver 31 can transmit and receive data to and from the tag reader device 20a via the network NW. The monitoring terminal device 30 may be located in proximity to the tag reader device 20a or may be located at a remote place.

The storage 32 stores, for example, data acquired by the tag reader device 20a from the radio tag 10b. The storage 32 corresponds to an acquired data storage of the present disclosure. The controller 33 includes, for example, a Central Processing Unit (CPU), Read Only Memory (ROM), Random Access Memory (RAM), an input interface, and an output interface, which are not illustrated. The CPU, the ROM, and the RAM (which are not illustrated) are connected to each other by an internal bus. The ROM stores programs such as BIOS. The CPU implements various functions by executing a program stored in the ROM or the storage 32 while using the RAM as a work area. The controller 33 can edit the data stored in the storage 32, such as classification and rearrangement. The power supply unit 34 supplies power to each component of the monitoring terminal device 30.

The alarm 37 outputs an alarm when the determination result obtained by the determination unit 15 of the radio tag 10b indicates that the determination result exceeds the predetermined threshold. That is, the alarm 37 outputs an alarm based on the determination result obtained by the determination unit 15. For example, when the determination result exceeds a predetermined threshold, an alarm is output by screen display on a display (not illustrated) or by output of a buzzer sound or the like from a speaker (not illustrated). With this configuration, the maintenance inspector can recognize the occurrence of temperature abnormality.

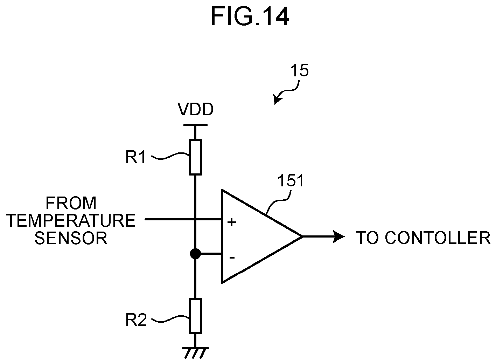

FIG. 14 is a diagram illustrating a configuration example of the determination unit 15 in FIG. 13. The determination unit 15 illustrated in FIG. 14 includes a comparator 151 and resistors R1 and R2. The comparator 151 has a positive input terminal (+) and a negative input terminal (-).

The resistors R1 and R2 are connected in series between a power supply voltage VDD and the ground potential. The connection point between the resistor R1 and the resistor R2 is connected to the negative input terminal of the comparator 151. A voltage value divided by the resistors R1 and R2 is input to the negative input terminal of the comparator 151. The comparator 151 outputs a voltage value corresponding to a result of comparison between the voltage value of the negative input terminal and the voltage value of the positive input terminal. That is, the comparator 151 outputs a high level voltage value (H) when the voltage value of the positive input terminal exceeds the voltage value of the negative input terminal. The comparator 151 outputs a low level voltage value (L) when the voltage value of the positive input terminal is equal to or less than the voltage value of the negative input terminal.

As described above, the determination unit 15 determines whether the detected value of the temperature output from the temperature sensor 12 (refer to FIG. 13) exceeds the threshold based on the voltage value obtained by resistive voltage dividing. Here, for example, the high-level voltage value (H) output by the comparator 151 is associated with "1", and the low-level voltage value (L) is associated with "0". Accordingly, the case where the data output from the determination unit 15 is "1" indicates that the temperature detected by the temperature sensor 12 exceeds the predetermined threshold. Referring back to FIG. 13, the data output from the determination unit 15 is stored in the storage 132. That is, the storage 132 stores the determination result obtained by the determination unit 15 and the identification information.

### (Example of Monitoring Target)

The monitoring target in the third embodiment is the same as the monitoring target described above with reference to FIGS. 2 and 3. However, the tag reader device 20a of the third embodiment is not fixed and moves in proximity to the mechanical device 200 (refer to FIG. 2). The tag reader device 20a moves across the positions where signal transmission and reception with the radio tag 10b are possible. That is, the tag reader device 20a circulates and acquires data from the radio tag 10b. The tag reader device 20a moves along a track such as a rail provided on a floor surface or a ceiling of a room in which the mechanical device 200 is installed, for example. In addition, the tag reader device 20a may move along a route set in advance on the floor surface, without the track. For example, a program that controls the motor 24 is stored in the storage 222 so as to move autonomously on a preset route. The controller 22 may then read and execute the program. The tag reader device 20a may be mounted on a drone, and the drone may move across the position where signal transmission and reception with the radio tag 10b are possible.

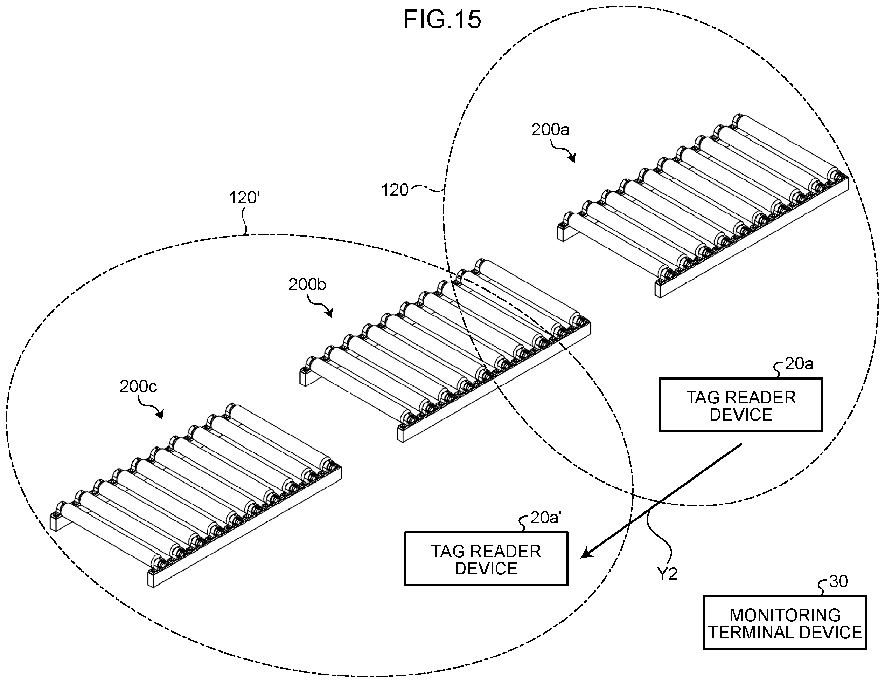

FIG. 15 is a diagram illustrating an example in which a plurality of the mechanical devices 200 are set as monitoring targets. In the example illustrated in FIG. 15, three mechanical devices 200a, 200b, and 200c are set as the monitoring targets. In the example illustrated in FIG. 5, one tag reader device 20a is provided for three mechanical devices 200a, 200b, and 200c. The tag reader device 20a moves in proximity to the three mechanical devices 200a, 200b, and 200c as indicated by arrow Y2, for example. In this example, reference numeral "20" is added to the tag reader device at the position before the movement, and reference numeral "20'" is added to the tag reader device at the position after the movement.

Here, in a communicable area 120 of the tag reader device 20a before the movement, all the mechanical components 40 included in the mechanical device 200a and some of the mechanical components 40 included in the mechanical device 200b are located. On the other hand, in a communicable area 120' of the tag reader device 20a' at the position after the movement, all the mechanical components 40 included in the mechanical device 200c and some of the mechanical components 40 included in the mechanical device 200b are located. In this manner, the tag reader device 20a moves such that all the mechanical components 40 are located in an area obtained by combining the communicable area 120 and the communicable area 120', making it possible for the tag reader device 20a to acquire the identification information and the temperature determination result from each radio tag 10b.

Referring back to FIG. 13, the identification information and the temperature determination result acquired from each radio tag 10b are stored in the storage 222 and then transmitted to the monitoring terminal device 30a via the network NW. The monitoring terminal device 30a stores the identification information and the temperature determination result in the storage 32. The controller 33 can edit the data stored in the storage 32, such as classification and rearrangement.

The detection of the temperature by the temperature sensor 12 of each radio tag 10b is performed, for example, at a predetermined interval. For example, the temperature sensor 12 detects the temperature once a day at a predetermined time. Alternatively, for example, the detection may be performed every preset time. For example, the temperature sensor 12 may detect the temperature every 1 hour, every 30 minutes, every 1 minute, and every 30 seconds.

The determination unit 15 determines whether the temperature detected by the temperature sensor 12 exceeds a predetermined threshold. The identification information and the temperature determination result are stored in the storage 132. As described above, the monitoring system can acquire the temperature determination result for each monitoring target identified by the acquired identification information, and monitor temperature abnormality.

### (Operation Example)

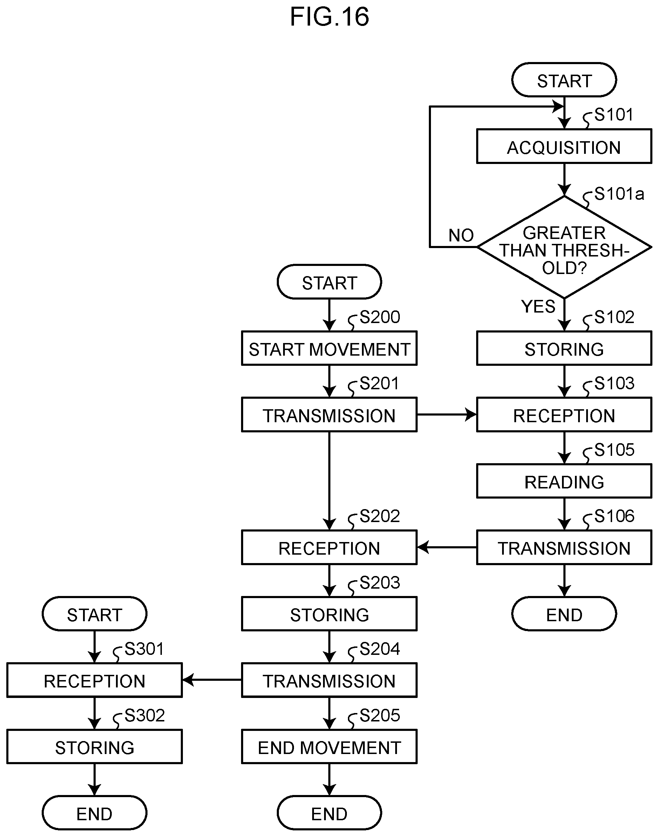

FIG. 16 is a flowchart illustrating an operation example of the monitoring system 100b according to the third embodiment. FIG. 16 illustrates operations of the radio tag 10b, the tag reader device 20a, and the monitoring terminal device 30a of the monitoring system 100b.

In FIG. 16, steps S101 to S106 illustrate an operation example of the radio tag 10b, steps S200 to S205 illustrate an operation example of the tag reader device 20a, and steps S301 to S302 illustrate an operation example of the monitoring terminal device 30a.

In FIG. 16, the radio tag 10b acquires temperature data from the temperature sensor 12 (step S101). Next, the radio tag 10b determines whether the temperature data exceeds a predetermined threshold (that is, whether the temperature data is larger than the threshold) using the determination unit 15 (step S101a). When the temperature data exceeds the predetermined threshold, data indicating that the temperature data exceeds the predetermined threshold is stored in the storage 132 as a temperature determination result (step S102). In a case where the temperature data does not exceed the predetermined threshold in step S101a, the process returns to step S101 and is continuously performed.

Thereafter, the tag reader device 20 starts movement (step S200). When a data read signal is transmitted from the tag reader device 20 to the radio tag 10b (step S201), the radio tag 10b receives the read signal (step S103). In response to this, the radio tag 10b reads the identification information stored in the storage 132 (step S105). The radio tag 10b transmits the temperature determination result together with the identification information (step S106), and the tag reader device 20 receives the temperature determination result and the identification information (step S202).

The tag reader device 20 stores the received temperature determination result and identification information in the storage 222 (step S203). Thereafter, the tag reader device 20 transmits the temperature determination result and the identification information (step S204), and the monitoring terminal device 30 receives the temperature determination result and the identification information (step S301). The tag reader device 20 ends the movement (step S205). The monitoring terminal device 30 stores the received temperature determination result and identification information in the storage 32 (step S302). The monitoring terminal device 30 can acquire the temperature determination result and the identification information by the above processing, and can edit the data stored in the storage 32, such as classification and rearrangement. The monitoring target can be monitored by utilizing the data stored in the storage 32. That is, the monitoring system can monitor the abnormality of the temperature for each monitoring target identified by the acquired identification information.

### (Example of Data to Be Acquired)

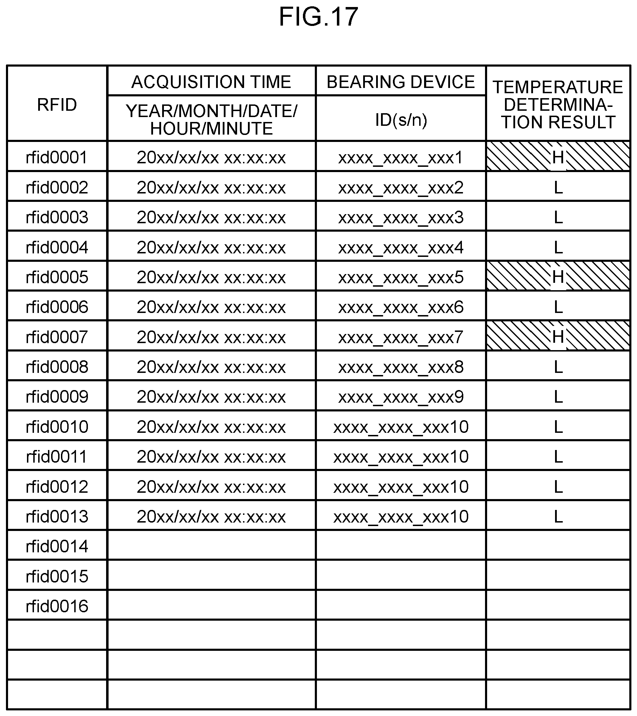

FIG. 17 is a diagram illustrating an example of data acquired by the tag reader device 20a from the radio tag 10b. FIG. 17 illustrates an example of data transmitted from the tag reader device 20a to the monitoring terminal device 30a and stored in the storage 32.

As illustrated in FIG. 17, identification information "rfid0001", "rfid0002",... regarding the RFID is stored in the storage 32 in association with other data items. For example, the identification information "rfid0001" is associated with acquisition time (namely, year/month/date/hour/minute), the serial number (s/n) as the identification information (ID) of the bearing, and the temperature determination result. The temperature determination result in this example is either "H" indicating that the temperature exceeds the predetermined threshold or "L" indicating that the temperature is equal to or less than the predetermined threshold. In the present example, the hatched portion in FIG. 17 is "H", which indicates that the determination result has exceeded the predetermined threshold.

### (Fourth Embodiment)

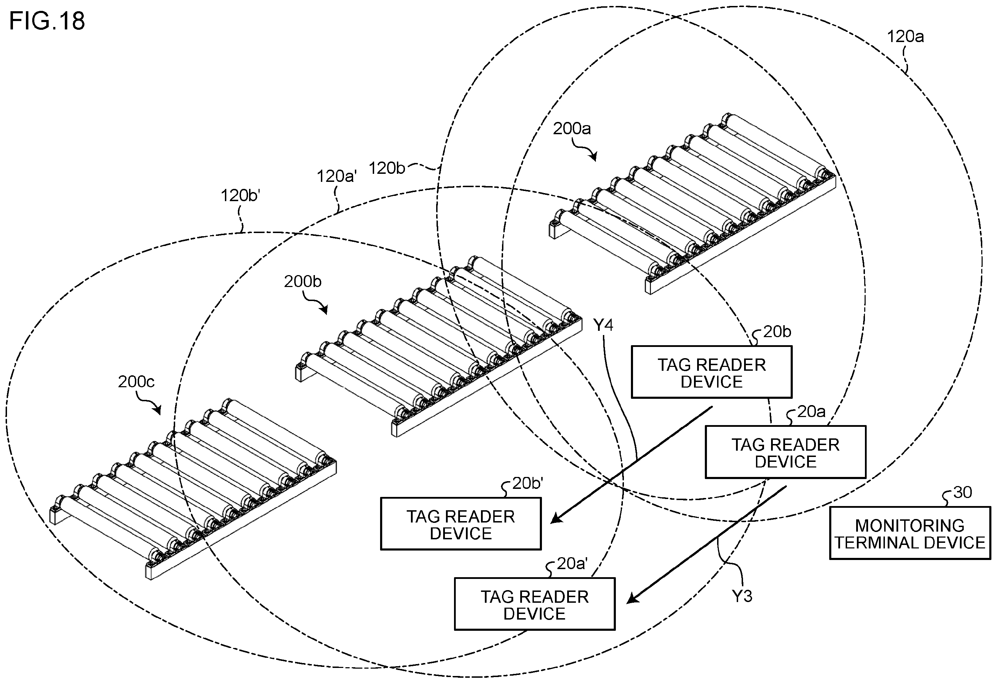

While the monitoring system according to the third embodiment described above uses one tag reader device 20, a plurality of tag reader devices may be used. FIG. 18 is a diagram illustrating a tag reader device of a monitoring system according to a fourth embodiment of the present disclosure. As illustrated in FIG. 18, the monitoring system according to the fourth embodiment uses two tag reader devices 20a and 20b. The tag reader device 20a moves in proximity to the three mechanical devices 200a, 200b, and 200c as indicated by arrow Y3, for example. In this example, reference numeral "20a" is added to the tag reader device at the position before the movement, and reference numeral "20a'" is added to the tag reader device at the position after the movement. Similarly, the tag reader device 20b moves in proximity to the three mechanical devices 200a, 200b, and 200c as indicated by arrow Y4, for example. In this example, reference numeral "20b" is added to the tag reader device at the position before the movement, and reference numeral "20b'" is added to the tag reader device at the position after the movement.

A communicable area 120a of the tag reader device 20a before movement includes all the mechanical components 40 included in the mechanical device 200a and some of the mechanical components 40 included in the mechanical device 200b. A communicable area 120b of the tag reader device 20b before movement includes all the mechanical components 40 included in the mechanical device 200a and some of the mechanical components 40 included in the mechanical device 200b. On the other hand, a communicable area 120a' of the tag reader device 20a' after the movement includes all the mechanical components 40 included in the mechanical device 200c and some of the mechanical components 40 included in the mechanical device 200b. A communicable area 120b' of the tag reader device 20b' after the movement includes all the mechanical components 40 included in the mechanical device 200c and some of the mechanical components 40 included in the mechanical device 200b. In this manner, the tag reader devices 20a and 20b are moved such that all the mechanical components 40 are provided in an area obtained by combining the communicable areas 120a and 120b before the movement and the communicable areas 120a' and 120b' after the movement, whereby the identification information and the temperature determination result can be acquired from each radio tag 10b by the tag reader devices 20a and 20b. The identification information and the temperature determination result acquired by the tag reader devices 20a and 20b are transmitted to the monitoring terminal device 30 and stored in the storage 32 in the monitoring terminal device 30. That is, the identification information and the temperature determination result are stored in the storage 32 provided to be shared by the tag reader devices 20a and 20b. The movements of the tag reader devices 20a and 20b make it possible to reliably acquire the identification information and the temperature determination result from each of the radio tags 10b of all the mechanical components 40. In particular, when the antennas of the tag reader devices 20a and 20b have mutually different directivities, the identification information and the temperature determination result can be reliably acquired by moving the tag reader devices.

According to the monitoring system of the third embodiment or the fourth embodiment described above, the production facility such as the site of the factory can be monitored without the need for the maintenance inspector to directly visit the place in proximity to the mechanical facility. Specifically, it is possible to detect a sign of abnormality associated with temperature change and identify the bearing. For example, temperature monitoring and processing can be performed in a remote control room. In each of the above embodiments, the bearing is a monitoring target, but the target is not limited to this and, for example, shaft extension by heat, motor rated operation, and the like can also be monitored. Furthermore, for example, in a case where a bearing and a box for packing the bearing are shipped together with a tag, it is also possible to monitor detection of an abnormal temperature during a period from shipment to assembly onto a mechanical device in the course of movement.

### (Fifth Embodiment)

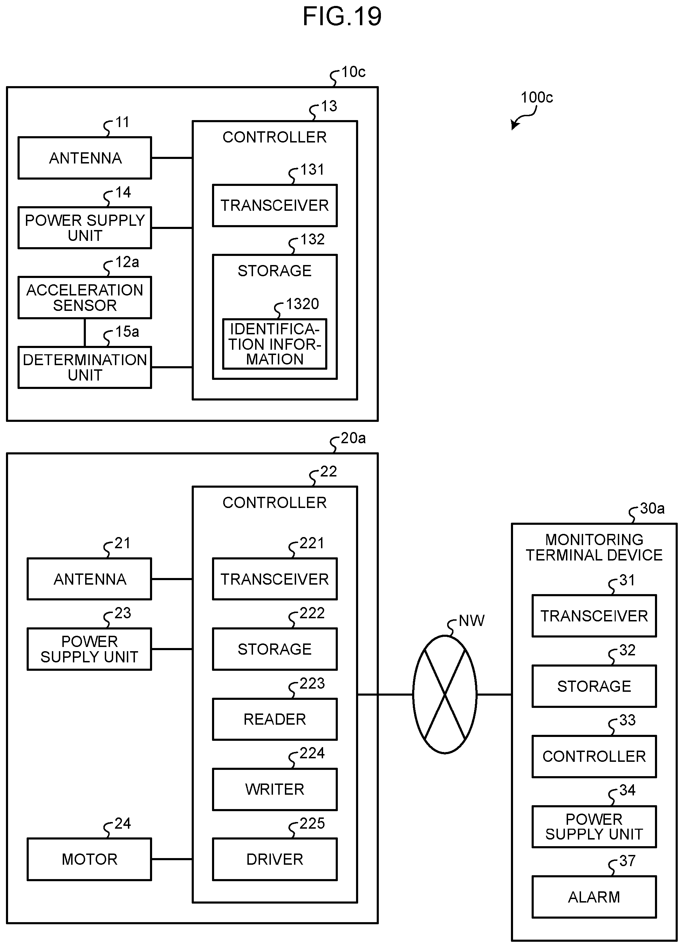

FIG. 19 is a diagram illustrating a monitoring system according to a fifth embodiment of the present disclosure. In FIG. 19, a monitoring system 100c includes a radio tag 10c, a tag reader device 20a, and a monitoring terminal device 30a. The radio tag 10c is provided on a monitoring target of the monitoring system 100c. The tag reader device 20a can acquire data from the radio tag 10c. In addition, the tag reader device 20a can write data into the radio tag 10c. The tag reader device 20a corresponds to a data acquisition device of the present disclosure.

### (Radio Tag)

The radio tag 10c includes an antenna 11, an acceleration sensor 12a, a controller 13, a power supply unit 14, and a determination unit 15a. The controller 13 includes a transceiver 131 and a storage 132. The radio tag 10c is an RFID tag, for example.

The antenna 11 is a transmission/reception antenna. That is, the antenna 11 has a function as a transmission antenna and a function as a reception antenna.

The acceleration sensor 12a detects acceleration. Specifically, the acceleration sensor 12a detects acceleration of the monitoring target on which the radio tag 10c is provided. The acceleration detected by the acceleration sensor 12a is transmitted to the determination unit 15a.

The transceiver 131 can wirelessly receive data via the antenna 11. The transceiver 131 can wirelessly transmit data via the antenna 11.

The storage 132 stores identification information 1320 for identifying the radio tag 10c itself. In addition, the storage 132 stores the determination result obtained by the determination unit 15a as data. The data stored in the storage 132 can be read.

The power supply unit 14 supplies power to each component in the radio tag 10c. The power supply unit 14 is a primary battery, for example. Since power is supplied from the power supply unit 14, the radio tag 10c can perform detection of the acceleration by the acceleration sensor 12a and store the vibration determination result based on the acceleration data in the storage 132.

The determination unit 15a determines whether the detection value of the acceleration output from the acceleration sensor 12a exceeds a predetermined threshold. The determination result obtained by the determination unit 15a is stored in the storage 132. For example, in a case where the detection value of the acceleration output by the acceleration sensor 12a exceeds the predetermined threshold, the data of the vibration determination result obtained by the determination unit 15a is "1", and in a case where the detection value is equal to or less than the predetermined threshold, the data of the vibration determination result obtained by the determination unit 15a is "0", and accordingly, "1" or "0" as the determination result is stored in the storage 132.

The threshold for the detection value of the acceleration is set as follows, for example. That is, the acceleration due to vibration at a time when the mechanical device 200 is first installed or the acceleration due to vibration at a later time when no abnormality occurs is measured in advance, and a value slightly exceeding the value of the measured acceleration is set as the threshold.

When a data read signal is transmitted from the tag reader device 20a, the radio tag 10c reads data stored in the storage 132 and transmits the data to the tag reader device 20a. At this time, the transceiver 131 of the radio tag 10c transmits the vibration determination result and the identification information to the tag reader device 20 in association with each other.

### (Tag Reader Device)

The tag reader device 20a includes an antenna 21, a controller 22, a power supply unit 23, and a motor 24.

The antenna 21 is a transmission/reception antenna. That is, the antenna 21 has a function as a transmission antenna and a function as a reception antenna.

The controller 22 includes a transceiver 221, a storage 222, a reader 223, a writer 224, and a driver 225. The transceiver 221 can wirelessly transmit and receive data to and from the radio tag 10c via the antenna 21. In addition, the transceiver 221 can transmit and receive data to and from the monitoring terminal device 30a via the network NW. The tag reader device 20a can transmit a vibration determination result based on acceleration data of the acceleration sensor 12a of the radio tag 10c to the monitoring terminal device 30a.

The storage 222 stores the data acquired by the transceiver 221. The storage 222 stores the vibration determination result and the identification information acquired by the transceiver 221 in association with each other. In addition, the storage 222 stores various data and programs needed for the operation of the tag reader device 20a.

The reader 223 can receive data transmitted from the radio tag 10c using the antenna 21 and the transceiver 221, and can read data stored in the radio tag 10c. With this configuration, the tag reader device 20a can acquire data from the radio tag 10c.

The reader 223 can perform wireless communication simultaneously with a plurality of the radio tags 10c, and can simultaneously acquire vibration determination results from the plurality of radio tags 10c. At this time, the vibration determination result associated with the identification information is acquired. Accordingly, the tag reader device 20a acquires the vibration determination result based on the acceleration data of each of the acceleration sensors of the plurality of radio tags 10c in a relatively short time. The tag reader device 20a transmits the vibration determination results based on acceleration data of a plurality of acceleration sensors to the monitoring terminal device 30a.

The writer 224 can transmit data to the radio tag 10c using the antenna 21 and the transceiver 221. With this configuration, the tag reader device 20a can write data into the radio tag 10c. The driver 225 controls the motor 24 to move the tag reader device 20a.

The power supply unit 23 supplies power to each component in the tag reader device 20a. The power supply unit 23 is a primary battery, for example.

### (Monitoring Terminal Device)

The monitoring terminal device 30a includes a transceiver 31, a storage 32, a controller 33, a power supply unit 34, and an alarm 37. The transceiver 31 can transmit and receive data to and from the tag reader device 20a via the network NW. The monitoring terminal device 30a may be located in proximity to the tag reader device 20a or may be located at a remote place.

The storage 32 stores, for example, data acquired by the tag reader device 20a from the radio tag 10c. The storage 32 corresponds to an acquired data storage of the present disclosure. The controller 33 includes, for example, a Central Processing Unit (CPU), Read Only Memory (ROM), Random Access Memory (RAM), an input interface, and an output interface, which are not illustrated. The CPU, the ROM, and the RAM (which are not illustrated) are connected to each other by an internal bus. The ROM stores programs such as BIOS. The CPU implements various functions by executing a program stored in the ROM or the storage 32 while using the RAM as a work area. The controller 33 can edit the data stored in the storage 32, such as classification and rearrangement. The power supply unit 34 supplies power to each component of the monitoring terminal device 30a.

The alarm 37 outputs an alarm when the determination result obtained by the determination unit 15 of the radio tag 10c indicates that the determination result exceeds the predetermined threshold. That is, the alarm 37 outputs an alarm based on the determination result obtained by the determination unit 15. For example, when the determination result exceeds a predetermined threshold, an alarm is output by screen display on a display (not illustrated) or by output of a buzzer sound or the like from a speaker (not illustrated). With this configuration, the maintenance inspector can recognize the occurrence of vibration exceeding the threshold.

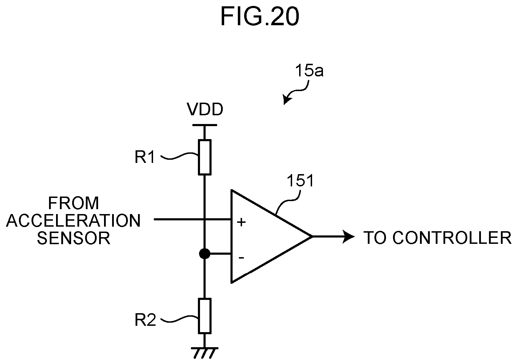

FIG. 20 is a diagram illustrating a configuration example of the determination unit 15a in FIG. 19. The determination unit 15a illustrated in FIG. 20 includes a comparator 151 and resistors R1 and R2. The comparator 151 has a positive input terminal (+) and a negative input terminal (-).

The resistors R1 and R2 are connected in series between a power supply voltage VDD and the ground potential. The connection point between the resistor R1 and the resistor R2 is connected to the negative input terminal of the comparator 151. A voltage value divided by the resistors R1 and R2 is input to the negative input terminal of the comparator 151. The comparator 151 outputs a voltage value corresponding to a result of comparison between the voltage value of the negative input terminal and the voltage value of the positive input terminal. That is, the comparator 151 outputs a high level voltage value (H) when the voltage value of the positive input terminal exceeds the voltage value of the negative input terminal. The comparator 151 outputs a low level voltage value (L) when the voltage value of the positive input terminal is equal to or less than the voltage value of the negative input terminal.

As described above, the determination unit 15a determines whether the detection value of the acceleration output from the acceleration sensor 12a (refer to FIG. 19) exceeds the threshold based on the voltage value obtained by resistive voltage dividing. Here, for example, the high-level voltage value (H) output by the comparator 151 is associated with "1", and the low-level voltage value (L) is associated with "0". Accordingly, the case where the data output from the determination unit 15a is "1" indicates that the vibration based on the acceleration detected by the acceleration sensor 12a exceeds the predetermined threshold. Referring back to FIG. 19, the data output from the determination unit 15a is stored in the storage 132. That is, the storage 132 stores the determination result obtained by the determination unit 15a and the identification information.

### (Example of Monitoring Target)

The monitoring target in the fifth embodiment is the same as the monitoring target described above with reference to FIGS. 2 and 3.

The tag reader device 20a is not fixed and moves in proximity to the mechanical device 200 (refer to FIG. 2). The tag reader device 20a moves across the positions where signal transmission and reception with the radio tag 10c are possible. That is, the tag reader device 20a circulates and acquires data from the radio tag 10c. The tag reader device 20a moves along a track such as a rail provided on a floor surface or a ceiling of a room in which the mechanical device 200 is installed, for example. In addition, the tag reader device 20a may move along a route set in advance on the floor surface, without the track. For example, a program that controls the motor 24 is stored in the storage 222 so as to move autonomously on a preset route. The controller 22 may then read and execute the program. The tag reader device 20a may be mounted on a drone, and the drone may move across the position where signal transmission and reception with the radio tag 10c are possible.

As described with reference to FIG. 15, a plurality of the mechanical devices 200 may be set as monitoring targets. The tag reader device 20a moves such that all the mechanical components 40 are located in an area obtained by combining the communicable area 120 and the communicable area 120', making it possible for the tag reader device 20a to acquire the identification information and the vibration determination result from each radio tag 10c.

Referring back to FIG. 19, the identification information and the vibration determination result acquired from each radio tag 10c are stored in the storage 222 and then transmitted to the monitoring terminal device 30a via the network NW. The monitoring terminal device 30a stores the identification information and the vibration determination result in the storage 32. The controller 33 can edit the data stored in the storage 32, such as classification and rearrangement.

The detection of the acceleration of each radio tag 10c by the acceleration sensor 12a is performed, for example, at a predetermined interval. For example, the acceleration sensor 12a detects acceleration once a day at a predetermined time. Alternatively, for example, the detection may be performed every preset time. For example, the acceleration sensor 12a may detect the acceleration every 1 hour, every 30 minutes, every 1 minute, and every 30 seconds.

The determination unit 15 determines whether the vibration based on the acceleration detected by the acceleration sensor 12a exceeds a predetermined threshold. The identification information and the vibration determination result are stored in the storage 132. As described above, the monitoring system can acquire the vibration determination result for each monitoring target identified by the acquired identification information, and monitor the occurrence of vibration exceeding the threshold.

### (Operation Example)

An operation example of the monitoring system 100c according to the fifth embodiment is similar to the monitoring system 100b according to the third embodiment described with reference to FIG. 16. An operation example of the monitoring system 100c will be described with reference to FIG. 16 again.

In FIG. 16, steps S101 to S106 illustrate an operation example of the radio tag 10c, steps S200 to S205 illustrate an operation example of the tag reader device 20a, and steps S301 to S302 illustrate an operation example of the monitoring terminal device 30a.

In FIG. 16, the radio tag 10c acquires acceleration data from the acceleration sensor 12a (step S101). Next, the radio tag 10 determines whether the vibration data based on the acceleration exceeds a predetermined threshold (that is, whether the vibration data is larger than the threshold) using the determination unit 15a (step S102). When the vibration data exceeds the predetermined threshold, data indicating that the vibration data exceeds the predetermined threshold is stored in the storage 132 as a vibration determination result (step S103). In a case where the vibration data does not exceed the predetermined threshold in step S102, the process returns to step S101 and is continuously performed.

Thereafter, the tag reader device 20a starts movement (step S200). When a data read signal is transmitted from the tag reader device 20a to the radio tag 10c (step S201), the radio tag 10c receives the read signal (step S104). In response to this, the radio tag 10c reads the identification information stored in the storage 132 (step S105). The radio tag 10c transmits the vibration determination result together with the identification information (step S106), and the tag reader device 20a receives the vibration determination result and the identification information (step S202).

The tag reader device 20a stores the received vibration determination result and identification information in the storage 222 (step S203). Thereafter, the tag reader device 20a transmits the vibration determination result and the identification information (step S204), and the monitoring terminal device 30a receives the vibration determination result and the identification information (step S301). The tag reader device 20a ends the movement (step S205). The monitoring terminal device 30a stores the received vibration determination result and identification information in the storage 32 (step S302). The monitoring terminal device 30a can acquire the vibration determination result and the identification information by the above processing, and can edit the data stored in the storage 32, such as classification and rearrangement. The monitoring target can be monitored by utilizing the data stored in the storage 32. That is, the monitoring system can monitor vibration for each monitoring target identified by the acquired identification information.

### (Example of Data to Be Acquired)

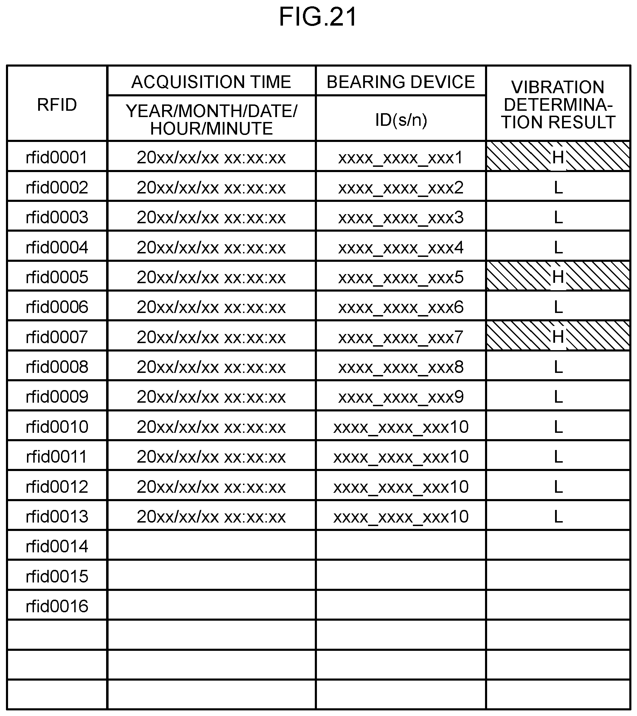

FIG. 21 is a diagram illustrating an example of data acquired by the tag reader device 20a from the radio tag 10c. FIG. 21 illustrates an example of data transmitted from the tag reader device 20a to the monitoring terminal device 30 and stored in the storage 32.

As illustrated in FIG. 21, identification information "rfid0001", "rfid0002",... regarding the RFID is stored in the storage 32 in association with other data items. For example, the identification information "rfid0001" is associated with acquisition time (namely, year/month/date/hour/minute), the serial number (s/n) as the identification information (ID) of the bearing, and the vibration determination result. The vibration determination result in this example is either "H" indicating that the data exceeds the predetermined threshold, or "L" indicating that the data is equal to or less than the predetermined threshold. In the present example, the hatched portion in FIG. 21 is "H", which indicates that the determination result has exceeded the predetermined threshold.

### (Sixth Embodiment)

While the monitoring system according to the fifth embodiment described above uses one tag reader device 20, a plurality of tag reader devices may be used. A monitoring system according to a sixth embodiment will be described with reference to FIG. 18 again. As illustrated in FIG. 18, the monitoring system according to the sixth embodiment uses two tag reader devices 20a and 20b. The tag reader device 20a moves in proximity to the three mechanical devices 200a, 200b, and 200c as indicated by arrow Y3, for example. In this example, reference numeral "20a" is added to the tag reader device at the position before the movement, and reference numeral "20a'" is added to the tag reader device at the position after the movement. Similarly, the tag reader device 20b moves in proximity to the three mechanical devices 200a, 200b, and 200c as indicated by arrow Y4, for example. In this example, reference numeral "20b" is added to the tag reader device at the position before the movement, and reference numeral "20b'" is added to the tag reader device at the position after the movement.

A communicable area 120a of the tag reader device 20a before movement includes all the mechanical components 40 included in the mechanical device 200a and some of the mechanical components 40 included in the mechanical device 200b. A communicable area 120b of the tag reader device 20b before movement includes all the mechanical components 40 included in the mechanical device 200a and some of the mechanical components 40 included in the mechanical device 200b. On the other hand, a communicable area 120a' of the tag reader device 20a' after the movement includes all the mechanical components 40 included in the mechanical device 200c and some of the mechanical components 40 included in the mechanical device 200b. A communicable area 120b' of the tag reader device 20b' after the movement includes all the mechanical components 40 included in the mechanical device 200c and some of the mechanical components 40 included in the mechanical device 200b. In this manner, the tag reader devices 20a and 20b are moved such that all the mechanical components 40 are provided in an area obtained by combining the communicable areas 120a and 120b before the movement and the communicable areas 120a' and 120b' after the movement, whereby the identification information and the vibration determination result can be acquired from each radio tag 10 by the tag reader devices 20a and 20b. The identification information and the vibration determination result acquired by the tag reader devices 20a and 20b are transmitted to the monitoring terminal device 30 and stored in the storage 32 in the monitoring terminal device 30. That is, the identification information and the vibration determination result are stored in the storage 32 provided to be shared by the tag reader devices 20a and 20b. The movements of the tag reader devices 20a and 20b make it possible to reliably acquire the identification information and the vibration determination result from each of the radio tags 10c of all the mechanical components 40. In particular, when the antennas of the tag reader devices 20a and 20b have mutually different directivities, the identification information and the vibration determination result can be reliably acquired by moving the tag reader devices.

According to the monitoring system of the fifth embodiment or the sixth embodiment described above, the production facility such as the site of the factory can be monitored without the need for the maintenance inspector to directly visit the place in proximity to the mechanical facility. Specifically, it is possible to detect a sign of abnormality associated with vibration change and identify the bearing. For example, vibration monitoring and processing can be performed in a remote control room. In a case where a bearing and a box used to pack the bearing are shipped together with a tag, for example, detection of abnormal vibration during a period from shipment to assembly onto a mechanical device can be monitored in the course of movement.

### (Seventh Embodiment)

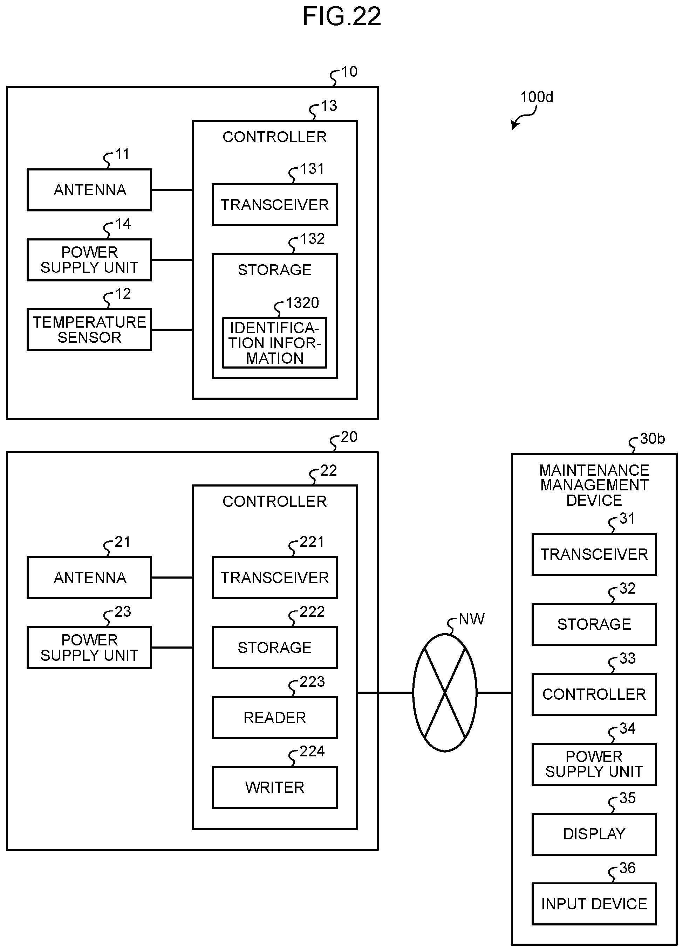

In the first to sixth embodiments described above, the monitoring system using the radio tag has been described. Hereinafter, a maintenance management system that performs maintenance management by using a radio tag will be described. FIG. 22 is a diagram illustrating a maintenance management system according to a seventh embodiment of the present disclosure. In FIG. 22, a maintenance management system 100d includes a radio tag 10, a tag reader device 20, and a maintenance management device 30b. The radio tag 10 is provided to a maintenance management target of the maintenance management system 100d. The tag reader device 20 can acquire data from the radio tag 10. In addition, the tag reader device 20 can write data into the radio tag 10. The tag reader device 20 corresponds to a data acquisition device of the present disclosure.

### (Radio Tag)

The radio tag 10 includes an antenna 11, a temperature sensor 12, a controller 13, and a power supply unit 14. The controller 13 includes a transceiver 131 and a storage 132. The radio tag 10 is an RFID tag, for example.

The antenna 11 is a transmission/reception antenna. That is, the antenna 11 has a function as a transmission antenna and a function as a reception antenna.

The temperature sensor 12 detects a temperature. Specifically, the temperature sensor 12 detects the temperature of the maintenance management target provided with the radio tag 10. The temperature detected by the temperature sensor 12 is stored in the storage 132 of the controller 13 as temperature data. That is, the temperature sensor 12 outputs temperature data corresponding to the temperature.

The transceiver 131 can wirelessly receive data via the antenna 11. The transceiver 131 can wirelessly transmit data via the antenna 11.

The storage 132 stores identification information 1320 for identifying the radio tag 10 itself. In addition, the storage 132 stores the temperature detected by the temperature sensor 12 as temperature data. The data stored in the storage 132 can be read.

The power supply unit 14 supplies power to each component in the radio tag 10. The power supply unit 14 is a primary battery, for example. Since power is supplied from the power supply unit 14, the radio tag 10 can perform detection of the temperature by the temperature sensor 12 and store temperature data in the storage 132.

When a data read signal is transmitted from the tag reader device 20, the radio tag 10 reads data stored in the storage 132 and transmits the read data to the tag reader device 20. At this time, the transceiver 131 of the radio tag 10 transmits the temperature data and the identification information to the tag reader device 20 in association with each other.

### (Tag Reader Device)

The tag reader device 20 includes an antenna 21, a controller 22, and a power supply unit 23.

The antenna 21 is a transmission/reception antenna. That is, the antenna 21 has a function as a transmission antenna and a function as a reception antenna.

The controller 22 includes a transceiver 221, a storage 222, a reader 223, and a writer 224. The transceiver 221 can wirelessly transmit and receive data to and from the radio tag 10 via the antenna 21. In addition, the transceiver 221 can transmit and receive data to and from the maintenance management device 30b via the network NW. The tag reader device 20 can transmit temperature data of the temperature sensor 12 of the radio tag 10 to the maintenance management device 30b.

The storage 222 stores the data acquired by the transceiver 221. The storage 222 stores the temperature data and the identification information acquired by the transceiver 221 in association with each other. In addition, the storage 222 stores various data and programs needed for the operation of the tag reader device 20.

The reader 223 can receive data transmitted from the radio tag 10 using the antenna 21 and the transceiver 221, and can read data stored in the radio tag 10. With this configuration, the tag reader device 20 can acquire data from the radio tag 10.

The reader 223 can perform wireless communication simultaneously with a plurality of the radio tags 10, and can simultaneously acquire temperature data from the plurality of radio tags 10. At this time, the temperature data associated with the identification information is acquired. Accordingly, the tag reader device 20 acquires temperature data of each of the temperature sensors of the plurality of radio tags 10 in a relatively short time. The tag reader device 20 transmits temperature data of a plurality of temperature sensors to the maintenance management device 30b.

The writer 224 can transmit data to the radio tag 10 using the antenna 21 and the transceiver 221. With this configuration, the tag reader device 20 can write data into the radio tag 10.

The power supply unit 23 supplies power to each component in the tag reader device 20. The power supply unit 23 is a primary battery, for example.

### (Maintenance Management Device)

The maintenance management device 30b includes a transceiver 31, a storage 32, a controller 33, a power supply unit 34, a display 35, and an input device 36. The transceiver 31 can transmit and receive data to and from the tag reader device 20 via the network NW. The maintenance management device 30b may be located in proximity to the tag reader device 20 or may be located at a remote location.

The storage 32 stores, for example, data acquired by the tag reader device 20 from the radio tag 10. The storage 32 corresponds to an acquired data storage of the present disclosure. The controller 33 includes, for example, a Central Processing Unit (CPU), Read Only Memory (ROM), Random Access Memory (RAM), an input interface, and an output interface, which are not illustrated. The CPU, the ROM, and the RAM (which are not illustrated) are connected to each other by an internal bus. The ROM stores programs such as BIOS. The CPU implements various functions by executing a program stored in the ROM or the storage 32 while using the RAM as a work area. The controller 33 can edit the data stored in the storage 32, such as classification and rearrangement. The controller 33 determines whether the temperature data stored in the storage 32 is equal to or greater than a predetermined threshold. As described below, the controller 33 has a function of a determination unit of the present disclosure. The power supply unit 34 supplies power to each component of the maintenance management device 30b.

The display 35 is a component that displays various data to a person who performs maintenance management. The display 35 can display data read from the storage 32 by the controller 33, for example. The input device 36 is a portion for a person who performs maintenance management to input data, and is a keyboard or a mouse, for example.

### (Example of Maintenance Management Target)

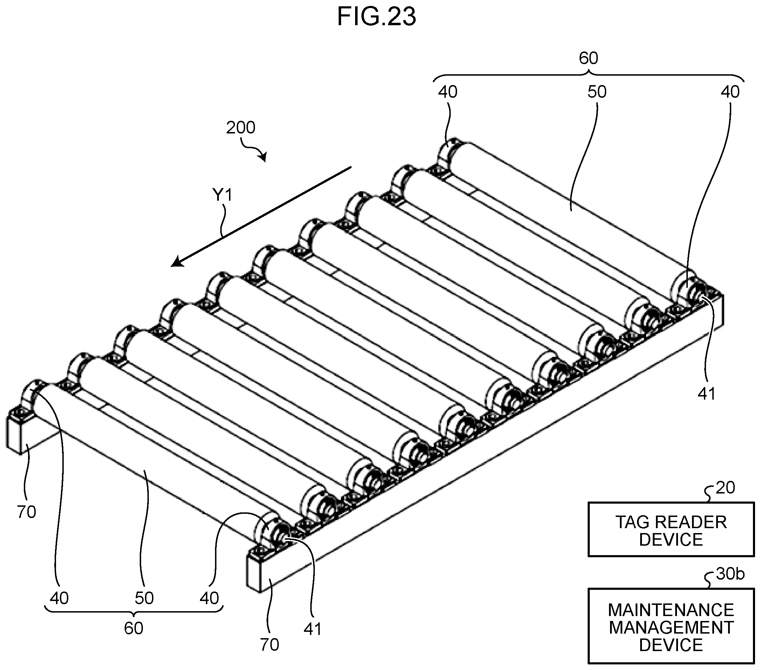

FIG. 23 is a diagram illustrating an example of a maintenance management target of the maintenance management system. FIG. 23 is a diagram illustrating a case where the mechanical device 200 is set as the maintenance management target. The mechanical component 40 in FIG. 23 is the same as the mechanical component 40 described with reference to FIG. 3.

The tag reader device 20 is not fixed and moves in proximity to the mechanical device 200. The tag reader device 20 moves across the positions where signal transmission and reception with the radio tag 10 are possible. That is, the tag reader device 20 circulates and acquires data from the radio tag 10. The tag reader device 20 moves along a track such as a rail provided on a floor surface or a ceiling of a room in which the mechanical device 200 is installed, for example. In addition, the tag reader device 20 may move along a route set in advance on the floor surface, without the track. For example, a motor is provided in the tag reader device 20, and a program that controls the motor so as to move autonomously on a preset route is stored in the storage 222. The controller 22 may read and execute the program.

Referring back to FIG. 22, the identification information and the temperature data acquired from each radio tag 10 are stored in the storage 222 and then transmitted to the maintenance management device 30b via the network NW. The maintenance management device 30b stores the identification information and the temperature data in the storage 32. The controller 33 can edit the data stored in the storage 32, such as classification and rearrangement.

The detection of the temperature by the temperature sensor 12 of each radio tag 10 is performed, for example, at a predetermined interval. For example, the temperature sensor 12 detects the temperature once a day at a predetermined time. Alternatively, for example, the detection may be performed every preset time. For example, the temperature sensor 12 may detect the temperature every 1 hour, every 30 minutes, every 1 minute, and every 30 seconds.

The identification information and the temperature data may be transmitted to the tag reader device 20 every time the temperature is detected by the radio tag 10, or may be collectively transmitted to the tag reader device 20 when the amount of data stored in the storage 132 of the radio tag 10 reaches a predetermined amount. In the former case, the maintenance management process can be performed more promptly. In the latter case, the consumption of the power supply unit 14 can be further reduced by collectively transmitting the data. As described above, the maintenance management system can acquire the temperature data for each maintenance management target identified by the acquired identification information, and monitor the abnormality of the temperature data.

### (Operation Example)

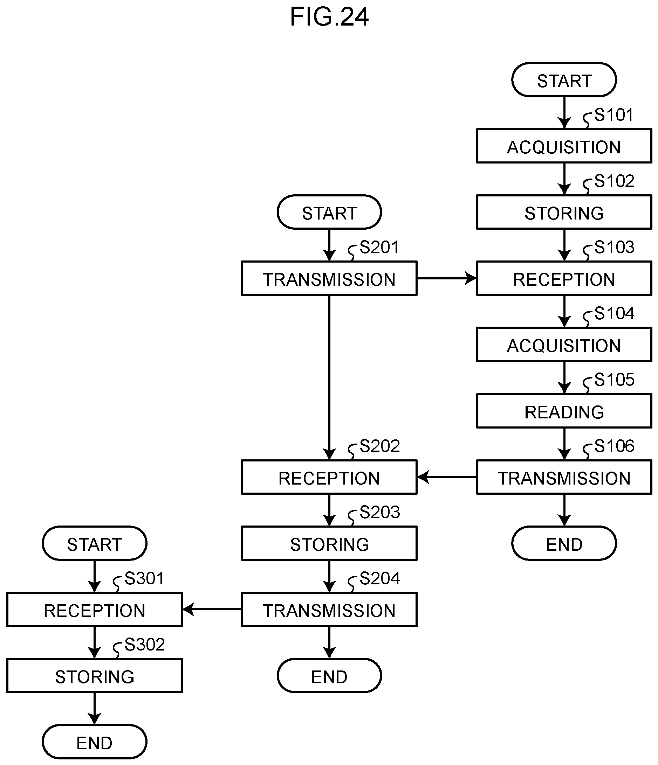

FIG. 24 is a flowchart illustrating an operation example of the maintenance management system 100d according to the seventh embodiment. FIG. 24 illustrates operations of the radio tag 10, the tag reader device 20, and the maintenance management device 30b of the maintenance management system 100d.

In FIG. 24, steps S101 to S106 illustrate an operation example of the radio tag 10, steps S201 to S204 illustrate an operation example of the tag reader device 20, and steps S301 to S302 illustrate an operation example of the maintenance management device 30b.

In FIG. 24, the radio tag 10 acquires temperature data from the temperature sensor 12 in advance (step S101) and stores the temperature data in the storage 132 (step S102).

Thereafter, when a data read signal is transmitted from the tag reader device 20 to the radio tag 10 (step S201), the radio tag 10 receives the read signal (step S103). Then, the radio tag 10 acquires temperature data from the temperature sensor 12 (step S104) and reads identification information stored in the storage 132 (step S105). The radio tag 10 transmits the temperature data together with the identification information (step S106), and the tag reader device 20 receives the temperature data and the identification information (step S202).

The tag reader device 20 stores the received temperature data and identification information in the storage 222 (step S203). Thereafter, the tag reader device 20 transmits the temperature data and the identification information (step S204), and the maintenance management device 30b receives the temperature data and the identification information (step S301). The maintenance management device 30b stores the received temperature data and identification information in the storage 32 (step S302). The maintenance management device 30b can acquire the temperature data and the identification information by the above processing, and can edit the data stored in the storage 32, such as classification and rearrangement. The maintenance management device 30b can monitor the maintenance management target by utilizing the data stored in the storage 32.

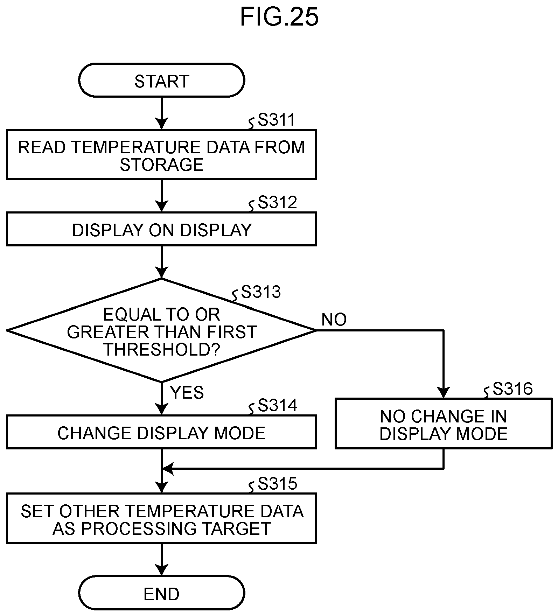

FIG. 25 is a flowchart illustrating a first example of processing in the controller 33 of the maintenance management device 30b in FIG. 22. The controller 33 performs the following processing by executing the program stored in the storage 32.

In FIG. 25, the controller 33 reads the temperature data stored in the storage 32 (step S311). The controller 33 displays the read temperature data on the display 35 (step S312). The controller 33 determines whether each temperature data value displayed on the display 35 is equal to or greater than a predetermined first threshold (step S313). When the result of the determination in step S313 indicates that the data is equal to or greater than the first threshold (Yes in step S313), a display mode change is performed on the temperature data (step S314). That is, anomalous data equal to or greater than the first threshold is displayed in a mode different from other temperature data. Thereafter, the above processing is repeated for other temperature data as processing targets (step S315).

On the other hand, when the result of the determination in step S313 indicates that the temperature is not equal to or greater than the first threshold (No in step S313), the display mode change is not performed on the temperature data (step S316). Thereafter, the above processing is repeated for other temperature data as processing targets (step S315).

As described above, by changing the display mode of the temperature data that is equal to or greater than the first threshold, the person who performs maintenance management can easily recognize the management target corresponding to the abnormal temperature data, and can promptly perform maintenance management such as repair and maintenance. Accordingly, the maintenance management system of the seventh embodiment makes it possible to monitor the abnormality of the temperature data for each maintenance management target identified by the acquired identification information.

### (Example of Temperature Data)

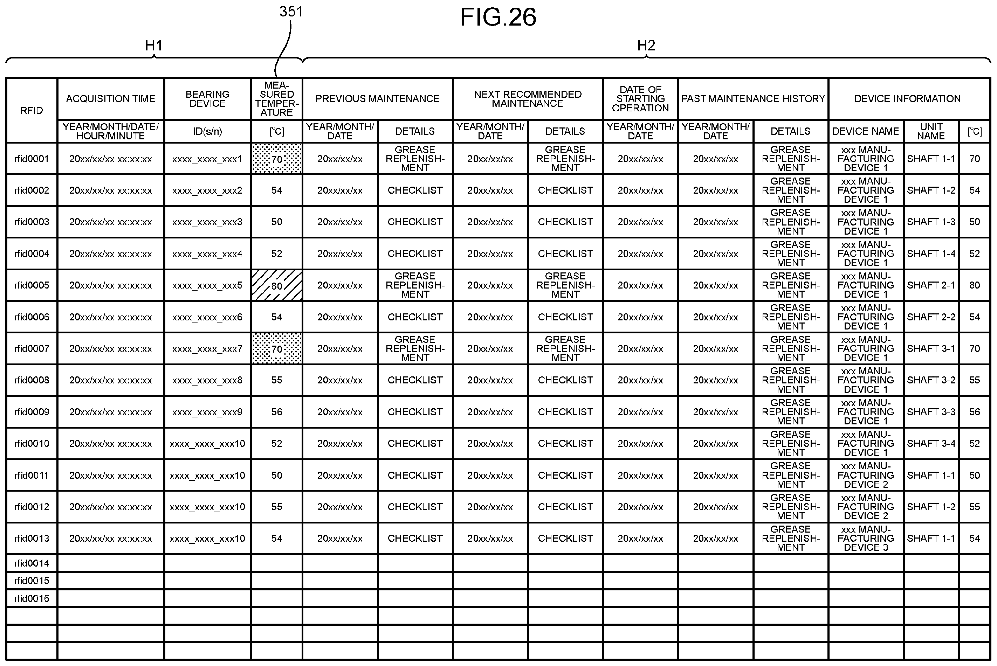

FIG. 26 is a diagram illustrating an example of data acquired from the radio tag 10 by the tag reader device 20. FIG. 26 illustrates temperature data transmitted from the tag reader device 20 to the maintenance management device 30b and stored in the storage 32. As illustrated in FIG. 26, the temperature data is displayed as a list on the screen of the display 35.

As illustrated in FIG. 26, identification information "rfid0001", "rfid0002",... regarding the RFID is stored in the storage 32 in association with other of data items. For example, the identification information "rfid0001" is associated with acquisition time (namely, year/month/date/hour/minute), the serial number (s/n) as the identification information (ID) of the bearing, and the measured temperature, that is, the temperature data. In this example, the identification information is further associated with the year/month/date and details of the previous maintenance (for example, grease replenishment, registration to checklist), the year/month/date and details of next recommended maintenance, the year/month/date of the operation start, the year/month/date and details of the past maintenance history, the device name as device information, the unit name, and the measured temperature.

Among the data items illustrated in FIG. 26, each data item (portion H1 in FIG. 26), including the identification information of the RFID, the acquisition time, the serial number of the bearing, and the measured temperature, is obtained from the radio tag 10. On the other hand, each data item (portion H2 in FIG. 26), including the year/month/date and details of the previous maintenance, the year/month/date and details of the next recommended maintenance, the year/month/date of the operation start date, the year/month/date and details of the past maintenance history, the device name as device information, the unit name, and the measured temperature, is used for maintenance management.

As described with reference to FIG. 25, the display mode change is performed on the measured temperature 351, which is the temperature data, among the data items illustrated in FIG. 26 based on the comparison determination result with respect to the first threshold. For example, the measured temperature equal to or higher than the first threshold is caused to be highlighted by changing the display color, increasing the display luminance, or using blinking display. The display mode change can attract attention of a person who performs maintenance management. In FIG. 26, a difference in display color is represented by, for example, shading.

In the example illustrated in FIG. 26, the display color is changed for the measured temperature of equal to or higher than 70°C. In the example illustrated in FIG. 26, the measured temperature of equal to or higher than 80°C, which is higher than 70°C, may be displayed in another display color. By preparing several types of first thresholds and performing comparison and determination using each threshold in step S313 in FIG. 25, warnings in plurality of stages can be achieved. For example, by displaying the temperature in yellow when the temperature is equal to or higher than 70°C and displaying the temperature in red when the temperature is equal to or higher than 80°C, the levels of the temperatures can be grasped intuitively.

The unit name is information that identifies the shaft member described with reference to FIG. 23. For example, these are "shaft 1-1", "shaft 1-2", "shaft 1-3", "shaft 1-4", "shaft 2-1", "shaft 2-2", "shaft 3-1", "shaft 3-2", "shaft 3-3", and "shaft 3-4".

The temperature measurement result of each shaft member can be displayed by a bar chart, for example, as described with reference to FIG. 7. That is, the maintenance management device 30b can edit each data item illustrated in FIG. 26 and display the data as a bar chart on a screen (not illustrated) as described above with reference to FIG. 7. In the bar chart illustrated in FIG. 7, temperature data values are displayed in a row. Since the data items of the bar chart are displayed in a row along the arrangement of the maintenance management targets, the person who performs the maintenance management can intuitively recognize the arrangement of the maintenance management targets. In the bar chart illustrated in FIG. 7, the display colors are varied for the case "shaft 1-1" and "shaft 3-1" at temperature equal to or higher than 70°C and for the case "shaft 2-1" at temperature equal to or higher than 80°C.

The temperature measurement result of each shaft member can be displayed as described above with reference to FIG. 8, for example. This point will be described with reference to FIG. 8 again. FIG. 8 is a diagram illustrating an example of a temperature measurement result of shaft 2-1. In the display state of FIG. 7, when the person who performs the maintenance management performs a predetermined operation, the controller 33 causes the display state to transition to the display state of FIG. 8. For example, when the mouse cursor is moved to the area of shaft 2-1 in the bar chart and a click is made on this area, the display state transitions to the display state of FIG. 8, which indicates details of the temperature data of shaft 2-1.

FIG. 8 illustrates an average value of temperature data for a certain period (for example, a whole day) for shaft 2-1. In the example illustrated in FIG. 8, temperature measurement is not performed on a non-operating day such as "May 1", and thus, there is no temperature data for the non-operating day. Referring to FIG. 8, it can be seen that the temperature of shaft 2-1 has increased on "May 8". With this configuration, a person who performs maintenance management can promptly perform maintenance management such as repair and maintenance works.

### (Eighth Embodiment)

The seventh embodiment described above performs determination to compare each temperature data value with the first threshold. The eighth embodiment described below performs the determination based on a difference in temperature between the temperature data values of the radio tags at the positions adjacent to each other. Other configurations and processing details are similar to the case of the seventh embodiment.

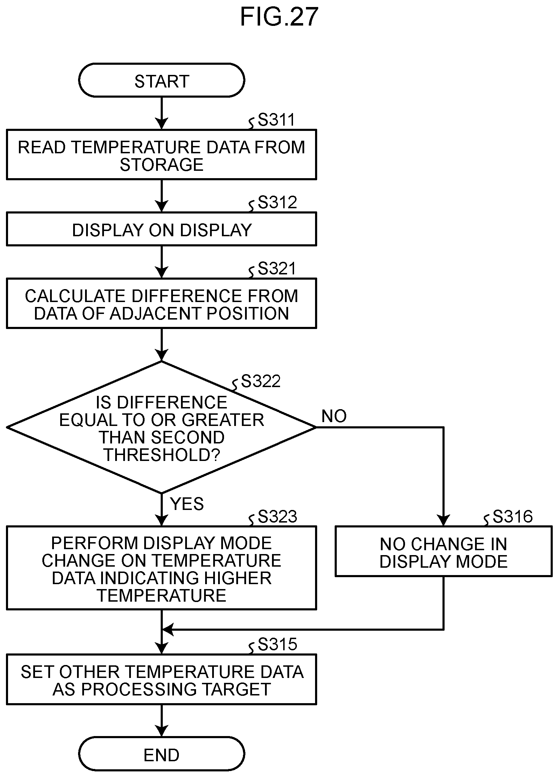

FIG. 27 is a flowchart illustrating a second example of processing in the controller 33 of the maintenance management device 30b in FIG. 22. The controller 33 performs the following processing by executing the program stored in the storage 32.

In FIG. 27, the controller 33 reads the temperature data stored in the storage 32 (step S311). The controller 33 displays the read temperature data on the display 35 (step S312). The controller 33 calculates a difference between the temperature data displayed on the display 35 and the temperature data of the radio tag at the adjacent position (step S321).

The controller 33 determines whether the difference calculated in step S321 is equal to or greater than a predetermined second threshold (step S322). When the result of the determination in step S322 indicates that the difference is equal to or greater than the second threshold (Yes in step S322), the display mode change is performed on the higher one among the temperature data values for which the difference has been calculated (step S323). That is, anomalous data having a difference equal to or greater than the second threshold is displayed in a mode different from other temperature data. Thereafter, the above processing is repeated for other temperature data as processing targets (step S315).

On the other hand, when the result of the determination in step S322 indicates that the difference is not equal to or greater than the second threshold (No in step S322), the display mode change is not performed on the temperature data (step S316). Thereafter, the above processing is repeated for other temperature data as processing targets (step S315).

As described above, when the difference between the temperature data values at positions adjacent to each other that is equal to or greater than the second threshold, the display mode change of the temperature data is performed, whereby the person who performs maintenance management can easily recognize the management target corresponding to the abnormal temperature data, and can promptly perform maintenance management such as repair and maintenance. Accordingly, the maintenance management system of the second embodiment makes it possible to monitor the abnormality of the temperature data for each maintenance management target identified by the acquired identification information.

### (Ninth Embodiment)

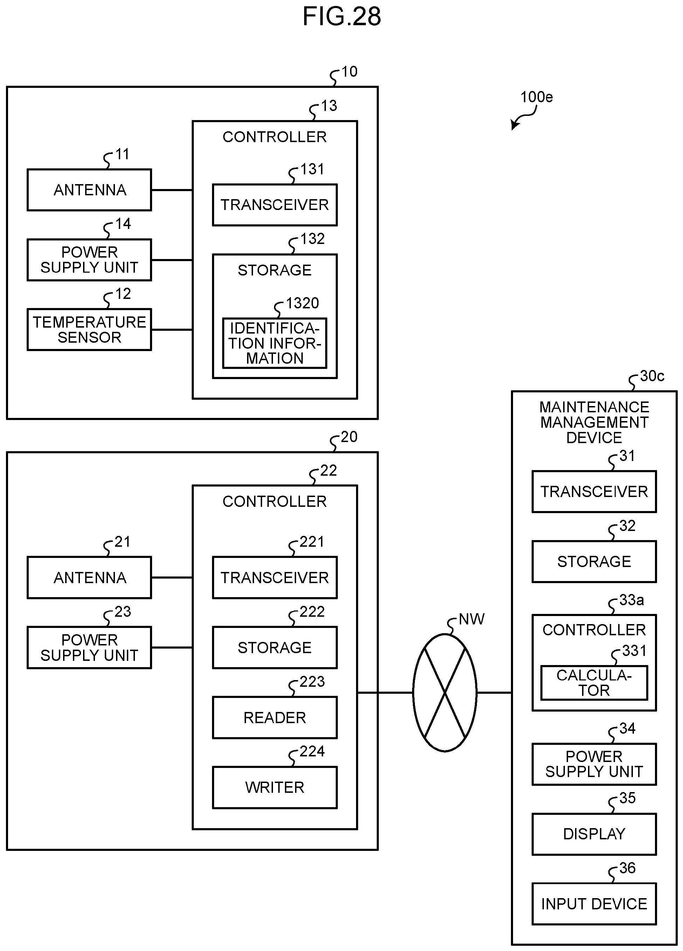

FIG. 28 is a diagram illustrating a maintenance management system according to a ninth embodiment of the present disclosure. In FIG. 28, in a maintenance management system 100e, a controller 33a of a maintenance management device 30c includes a calculator 331. The calculator 331 calculates an average value of the temperature data. In the seventh embodiment described above, determination to compare each temperature data value with the first threshold is performed. In the maintenance management system 100e of the ninth embodiment, the determination is performed based on the difference from the average value of the temperature data values. Other configurations and processing details are similar to the case of the seventh embodiment.

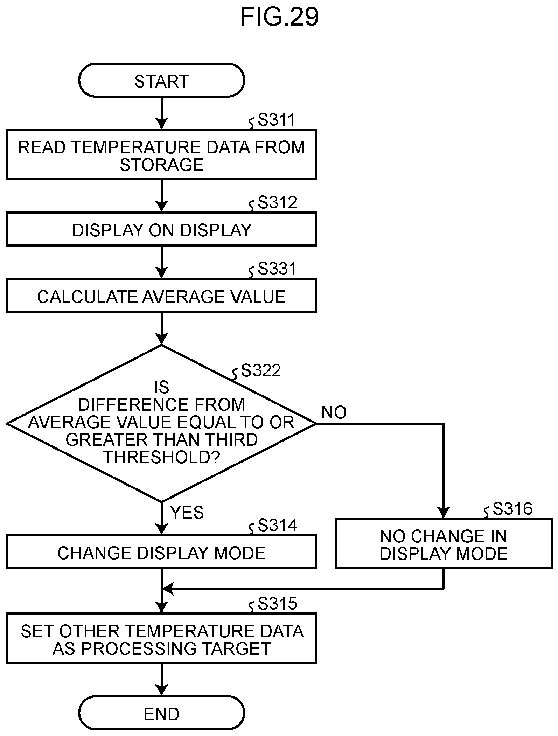

FIG. 29 is a flowchart illustrating a third example of processing in the controller 33 of the maintenance management device 30c in FIG. 28. The controller 33 performs the following processing by executing the program stored in the storage 32.

In FIG. 29, the controller 33 reads the temperature data values stored in the storage 32 (step S311). The controller 33 displays the read temperature data values on the display 35 (step S312). The controller 33 calculates an average value of temperature data values displayed on the display 35 (step S331). The processing of calculating the average value in step S331 corresponds to the processing performed by the calculator of the present disclosure.

The controller 33 determines whether the difference from the average value calculated in step S331 is equal to or greater than a predetermined third threshold (step S332). When the result of the determination in step S332 indicates that the difference from the average value is equal to or greater than the third threshold (Yes in step S332), the display mode change is performed on the temperature data value (step S314). That is, anomalous data having a difference equal to or greater than the third threshold is displayed in a mode different from the other temperature data values. Thereafter, the above processing is repeated for other temperature data values as processing targets (step S315).

On the other hand, when the result of the determination in step S322 indicates that the difference from the average value is not equal to or greater than the third threshold (No in step S332), the display mode change is not performed on the temperature data value (step S316). Thereafter, the above processing is repeated for other temperature data values as processing targets (step S315).

As described above, by performing the display mode change on the temperature data value of which the difference from the average value of the temperature data values is equal to or greater than the third threshold, the temperature data value deviating from the average value can be easily extracted. With this configuration, the person who performs maintenance management can easily recognize the management target corresponding to the abnormal temperature data and can promptly perform maintenance management such as repair and maintenance. Accordingly, the maintenance management system of the third embodiment makes it possible to monitor the abnormality of the temperature data for each maintenance management target identified by the acquired identification information.

### (Modification)

In each of the above embodiments, the temperature data is displayed by a bar chart, but may be displayed in another display format. For example, the temperature data may be displayed by a pie chart or a line chart.

### Reference Signs List

1-1 to 1-4, 2-1, 2-2, 3-1 to 3-4 SHAFT
10, 10a, 10b, 10c RADIO TAG
11, 21 ANTENNA
12 TEMPERATURE SENSOR
12a ACCELERATION SENSOR
13, 22, 33, 33a CONTROLLER
14, 23, 34 POWER SUPPLY UNIT
15, 15a DETERMINATION UNIT
20, 20a, 20a', 20b, 20b' TAG READER DEVICE
24 MOTOR
30, 30a MONITORING TERMINAL DEVICE
30b, 30c MAINTENANCE MANAGEMENT DEVICE
31, 131, 221 TRANSCEIVER
32, 132, 222 STORAGE
35 DISPLAY
36 INPUT DEVICE
37 ALARM
40 MECHANICAL COMPONENT
41 SHAFT MEMBER
42 BEARING
43 THROUGH HOLE
44a, 44b HOLE
50 ROLLER MEMBER
60 ROLLER DEVICE
70 SUPPORT BASE
100, 100a, 100b, 100c MONITORING SYSTEM
100d, 100e MAINTENANCE MANAGEMENT SYSTEM
151 COMPARATOR
200, 200a, 200b, 200c MECHANICAL DEVICE
223 READER
224 WRITER
225 DRIVER
331 CALCULATOR
351 MEASURED TEMPERATURE
1320 IDENTIFICATION INFORMATION
R1, R2 RESISTOR

## Claims

1. A monitoring system comprising: a radio tag provided to a monitoring target; and a data acquisition device configured to acquire data from the radio tag,
wherein the radio tag comprises:
a storage configured to store identification information; and
a physical quantity sensor configured to detect a physical quantity of the monitoring target and output physical quantity data corresponding to the detected physical quantity, and
the monitoring system is configured to monitor the monitoring target based on the physical quantity data and the identification information acquired by the data acquisition device.

2. The monitoring system according to claim 1,
wherein the physical quantity is a temperature of the monitoring target, and
the physical quantity sensor is a temperature sensor configured to detect a temperature of the monitoring target and output temperature data corresponding to the detected temperature,
the monitoring system further comprises a transceiver configured to transmit the temperature data output from the temperature sensor and the identification information, and
the monitoring system is configured to monitor the monitoring target based on the temperature data and the identification information acquired by the data acquisition device.

3. The monitoring system according to claim 2,
wherein the data acquisition device is fixed in proximity to the monitoring target.

4. The monitoring system according to claim 3, further comprising
a plurality of the data acquisition devices,
wherein the radio tag is provided to each of a plurality of the monitoring targets.

5. The monitoring system according to any one of claims 2 to 4, wherein the transceiver is configured to transmit the temperature data and the identification information in association with each other.

6. The monitoring system according to any one of claims 2 to 4,
wherein the radio tag is provided to a bearing,
the monitoring system further comprises a monitoring terminal device for monitoring the bearing, and
the monitoring terminal device comprises an acquired data storage configured to store data acquired by the data acquisition device, and the monitoring terminal device is configured to monitor the bearing based on the data stored in the acquired data storage.

7. The monitoring system according to claim 1,
wherein the physical quantity is a temperature of the monitoring target,
the physical quantity sensor is a temperature sensor configured to detect the temperature of the monitoring target and output temperature data corresponding to the detected temperature,
the monitoring system further comprises:
a determination unit configured to determine whether the temperature detected by the temperature sensor exceeds a predetermined threshold;
a storage configured to store a determination result and identification information, the determination result having been obtained by the determination unit and indicating that the temperature detected by the temperature sensor exceeds the predetermined threshold; and
a transceiver configured to transmit the determination result and the identification information,
the data acquisition device is configured to move in proximity to the radio tag, and
the monitoring system is configured to monitor the monitoring target based on the determination result and the identification information acquired from the radio tag by the data acquisition device when the data acquisition device moves in proximity to the radio tag.

8. The monitoring system according to claim 7, further comprising
a plurality of the data acquisition devices,
wherein the radio tag is provided to each of a plurality of the monitoring targets, and
the data acquisition devices are configured to store the identification information acquired from the radio tags when the data acquisition devices move in proximity to the radio tags.

9. The monitoring system according to claim 7 or 8, wherein the transceiver is configured to transmit the determination result and the identification information in association with each other.

10. The monitoring system according to claim 7 or 8,
wherein the radio tag is provided to a bearing,
the monitoring system further comprises a monitoring terminal device for monitoring the bearing, and
the monitoring terminal device comprises an acquired data storage configured to store data acquired by the data acquisition device, and the monitoring terminal device is configured to monitor the bearing based on the data stored in the acquired data storage.

11. The monitoring system according to claim 10,
wherein the monitoring terminal device further comprises an alarm configured to output an alarm based on the determination result.

12. The monitoring system according to claim 1,
wherein the physical quantity is acceleration of the monitoring target,
the physical quantity sensor is an acceleration sensor configured to detect the acceleration of the monitoring target,
the monitoring system further comprises:
a determination unit configured to determine whether vibration based on the acceleration detected by the acceleration sensor exceeds a predetermined threshold;
a storage configured to store a determination result and identification information, the determination result having been obtained by the determination unit and indicating that the vibration based on the acceleration detected by the acceleration sensor exceeds the predetermined threshold; and
a transceiver configured to transmit the determination result and the identification information,
the data acquisition device is configured to move in proximity to the radio tag, and
the monitoring system is configured to monitor the monitoring target based on the determination result and the identification information acquired from the radio tag by the data acquisition device when the data acquisition device moves in proximity to the radio tag.

13. The monitoring system according to claim 12, further comprising
a plurality of the data acquisition devices,
wherein the radio tag is provided to each of a plurality of the monitoring targets, and
the data acquisition devices are configured to store the identification information acquired from the radio tags when the data acquisition devices move in proximity to the radio tags.

14. The monitoring system according to claim 12 or 13, wherein the transceiver is configured to transmit the determination result and the identification information in association with each other.

15. The monitoring system according to claim 12 or 13,
wherein the radio tag is provided to a bearing,
the monitoring system further comprises a monitoring terminal device for monitoring the bearing, and
the monitoring terminal device comprises an acquired data storage configured to store data acquired by the data acquisition device, and the monitoring terminal device is configured to monitor the bearing based on the data stored in the acquired data storage.

16. The monitoring system according to claim 15, wherein the monitoring terminal device further comprises an alarm configured to output an alarm based on the determination result.

17. A maintenance management system comprising:
a plurality of radio tags provided in proximity to a plurality of maintenance management targets and each comprising a temperature sensor configured to acquire a temperature of the corresponding maintenance management target;
a data acquisition device configured to acquire f temperature data values from the radio tags;
a storage configured to store the temperature data values acquired by the data acquisition device;
a determination unit configured to determine whether the temperature data value stored in the storage is equal to or greater than a predetermined threshold; and
a display configured to display the temperature data values read from the storage,
wherein, based on a determination result obtained by the determination unit, the display displays a temperature data value indicating the temperature equal to or higher than the predetermined threshold, among the temperature data values, in a mode different from the other temperature data values.

18. The maintenance management system according to claim 17,
wherein, based on a determination result obtained by the determination unit, when the temperature data value indicates a value equal to or greater than a first threshold, the display displays the temperature data value in a mode different from the other temperature data values.

19. The maintenance management system according to claim 17,
wherein, based on a determination result obtained by the determination unit, when a difference between the temperature data values of maintenance management targets at adjacent positions is equal to or greater than a second threshold, the display displays the higher temperature data value thereof in a mode different from the other temperature data value thereof.

20. The maintenance management system according to claim 17, further comprising
a calculator configured to calculate an average value,
wherein, based on a determination result obtained by the determination unit, when a difference between the average value and the temperature data value is equal to or greater than a third threshold, the display displays the temperature data value in a mode different from the other temperature data values.

21. The maintenance management system according to any one of claims 17 to 20,
wherein the display is configured to display the temperature data values by a bar chart, and
among the temperature data values to be displayed by the bar chart, the temperature data value to be displayed in a mode different from the other temperature data values is displayed in a color different from a display color of the other temperature data values.
